# EUROPÄISCHE PATENTSCHRIFT

(11) **EP 2 278 965 B1**
(45) Veröffentlichungstag und Bekanntmachung des Hinweises auf die Patenterteilung: **13.11.2013**
(21) Anmeldenummer: 09722891.0
(22) Anmeldetag: 14.03.2009
(51) Int. Cl.: A61K 31/4155, C07D 231/10, A61P 31/18

(54) **SUBSTITUIERTE PYRAZOLAMIDE UND IHRE VERWENDUNG**
SUBSTITUTED PYRAZOLAMIDES AND THE USE THEREOF
PYRAZOLAMIDES SUBSTITUÉS ET LEUR UTILISATION

(30) Priorität: 17.03.2008 DE 102008015032
(43) Veröffentlichungstag der Anmeldung: 02.02.2011
(73) Patentinhaber: AiCuris GmbH & Co. KG, 42117 Wuppertal (DE)
(72) Erfinder: SCHOHE-LOOP, Rudolf, 42327 Wuppertal (DE); WELKER, Reinhold, 64625 Bensheim (DE); PAESSENS, Arnold, 42781 Haan (DE); BAUSER, Marcus, 10439 Berlin (DE); STOLL, Friedrike, 40215 Düsseldorf (DE); DITTMER, Frank, 40627 Düsseldorf (DE); HENNINGER, Kerstin, 42289 Wuppertal (DE); PAULSEN, Daniela, 42105 Wuppertal (DE); LANG, Dieter, 42553 Velbert (DE)
(74) Vertreter: Kilger, Ute
(86) Internationale Anmeldenummer: PCT/EP2009/001877
(87) Internationale Veröffentlichungsnummer: WO 2009/115252

(56) Entgegenhaltungen:
- EP-A- 0 112 776
- WO-A-2005/000820
- WO-A-2006/065209
- WO-A-2006/099231
- "ZINC04827711 [2,5-bis(4-fluorophenyl)pyrazol-3-yl]piper idin-1-ylmethanone"[Online] 12. September 2005 (2005-09-12), XP002530071 Gefunden im Internet: URL:http://pubchem.ncbi.nlm.nih.gov> [gefunden am 2009-05-29]
- "CID3315199 [2-(3-methylphenyl)-5-phenylpyrazol-3-yl]- (4-methylpiperidin-1-yl)"[Online] 7. September 2005 (2005-09-07), XP002530072 Gefunden im Internet: URL:http://pubhcem.ncbi.nlm.nih.gov> [gefunden am 2009-05-29]
- "ZINC04560769 [2-(4-fluorophenyl)-5-phenylpyrazol-3-yl]- morpholin-4-ylmethanone"[Online] 18. September 2005 (2005-09-18), XP002530073 Gefunden im Internet: URL:http://pubchem.ncbi.nlm.nih.gov> [gefunden am 2009-05-29]
- "ZINC04374875 1-[4-[2-(4-methylphenyl)-5-phenylpyrazole- 3-carbonyl]piperazin-1-yl]ethanone"[Online ] 13. September 2005 (2005-09-13), XP002530074 Gefunden im Internet: URL:http://pubchem.ncbi.nlm.nih.gov> [gefunden am 2009-05-29]
- "ZINC04908325 [2-(2,4-dimethylphenyl)-5-(2-fluorophenyl) pyrazol-3-yl]-(4-methylpiperidin-1-yl)meth anone"[Online] 14. September 2005 (2005-09-14), XP002530075 Gefunden im Internet: URL:http://pubchem.ncbi.nlm.nih.gov> [gefunden am 2009-04-29]
- "ZINC04407915 4-[2-(4-fluorophenyl)-5-phenylpyrazole-3-c arbonyl]piperazine-1-ethylcarboxylate"[Onl ine] 18. September 2005 (2005-09-18), XP002530076 Gefunden im Internet: URL:http://pubchem.ncbi.nlm.nih.gov> [gefunden am 2009-05-29]
- GENIN M J ET AL: "NOVEL 1,5-DIPHENYLPYRAZOLE NONNUCLEOSIDE HIV-1 REVERSE TRANSCRIPTASE INHIBITORS WITH ENHANCED ACTIVITY VERSUS THE DELAVIRDINE-RESISTANT P236L MUTANT: LEAD IDENTIFICATION AND SAR OF 3- AND 4-SUBSTITUTED DERIVATIVES" JOURNAL OF MEDICINAL CHEMISTRY, AMERICAN CHEMICAL SOCIETY, WASHINGTON, US, Bd. 43, 1. Januar 2000 (2000-01-01), Seiten 1034-1040, XP002178918 ISSN: 0022-2623

## Beschreibung

Die vorliegende Erfindung betrifft neue substituierte Pyrazolamide, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von retroviralen Erkrankungen, bei Menschen und/oder Tieren.

HIV (Virus der humanen Immundefizienz) verursacht eine chronisch-persistente, progrediente Infektion. Die Erkrankung verläuft über verschiedene Stadien von der asymptomatischen Infektion bis zum Krankheitsbild AIDS (*Acquired Immunodeficiency Syndrom*). AIDS ist das finale Stadium der durch Infektion hervorgerufenen Erkrankung. Charakteristisch für die HIV/AIDS-Erkrankung ist die lange klinische Latenzzeit mit persistierender Virämie, die im Endstadium zum Versagen der Immunabwehr führt.

Durch die Einführung der anti-HIV Kombinationstherapie gelang es dann in den 90-er Jahren, die Krankheitsprogression nachhaltig zu verlangsamen und damit die Lebenserwartung HIV-infizierter Patienten substantiell zu verlängern (Palella et al., N. Engl. J. Med. 1998, 238, 853-860).

Die derzeit auf dem Markt befindlichen anti-HIV-Substanzen hemmen die Replikation des HI-Virus durch Inhibition der essentiellen viralen Enzyme Reverse Transkriptase (RT), der Protease oder der HIV-Fusion (Übersicht in Richman, Nature 2001, 410, 995-1001). Es existieren zwei Klassen von RT-Inhibitoren: Nukleosidische RT-Inhibitoren (NRTI) wirken durch kompetetive Inhibition oder Kettenabbruch bei der DNA-Polymerisation. Nicht-Nukleosidische RT-Inhibitoren (NNRTI) binden allosterisch an einer hydrophoben Tasche in der Nähe des aktiven Zentrums der RT und vermitteln eine Konformationsänderung des Enzyms. Die derzeit verfügbaren Proteaseinhibitoren (PI) hingegen blockieren das aktive Zentrum der viralen Protease und verhindern somit die Reifung neuentstehender Partikel zu infektiösen Virionen.

Da die Monotherapie mit den momentan verfügbaren anti-HIV-Medikamenten innerhalb sehr kurzer Zeit zum Therapieversagen durch Selektion resistenter Viren führt, erfolgt üblicherweise eine Kombinationstherapie mit mehreren anti-HIV-Substanzen aus verschiedenen Klassen (*highly active antiretroviral therapy* = HAART; Carpenter et al., J. Am. Med. Assoc. 2000, 283, 381-390).

Trotz der Fortschritte in der antiretroviralen Chemotherapie zeigen neuere Untersuchungen, dass mit den zur Verfügung stehenden Medikamenten eine Eradikation von HIV und damit verbunden eine Heilung der HIV-Infektion nicht zu erwarten ist: latentes Virus verbleibt in ruhenden Lymphozyten und stellt ein Reservoir für eine Reaktivierung und damit für eine erneute Virusausbreitung dar (Finzi et al., Nature Med. 1999, 5, 512-517; Ramratnam et al., Nature Med. 2000, 6, 82-85). HIV-infizierte Patienten sind daher zeitlebens auf eine effiziente antivirale Therapie angewiesen. Trotz Kombinationstherapie kommt es nach einiger Zeit zur Selektion resistenter Viren. Da für jede therapeutische Klasse charakteristische Resistenzmutationen akkumulieren, bedeutet das Versagen einer Therapie oft einen Wirkverlust der kompletten Substanzklasse. Diese Kreuzresistenzproblematik ist bei der Klasse der NNRTIs am ausgeprägtesten, da hier schon eine einzelne Punktmutation in der RT ausreichen kann, um einen Wirkverlust aller NNRTIs zu bewirken (Übersicht in Kavlick & Mitsuya, Antiretroviral Chemotherapy (Hrsg. De Clercq E.), 2001, ASM Press, 279-312).

Begünstigt wird die Entstehung von Resistenzen meist durch die schlechte Compliance der Patienten, die durch ein ungünstiges Nebenwirkungsprofil und kompliziertes Dosierungsschema der anti-HIV-Medikamente hervorgerufen wird.

Somit besteht ein dringender Bedarf nach neuen therapeutischen Optionen zur Bekämpfung der HIV-Infektion. Dazu ist die Identifizierung neuer chemischer Leitstrukturen bedeutsam und ein vordringliches Ziel der Therapieforschung zu HIV, die entweder ein neues Target in der Vermehrung des HIV adressieren und/oder gegen die wachsende Zahl resistenter klinischer HIV-Isolate wirksam sind.

US 5,624,941 und EP 576357 beschreiben Pyrazole als Cannabinoid-Rezeptor-Antagonisten, EP 418845, EP 554829 und WO 04/050632 unter anderem zur Behandlung von inflammatorischen und thrombotischen Erkrankungen, WO 03/037274 als Natriumionen-Kanal-Inhibitoren zur Behandlung von Schmerz, WO 06/015860 als Adenosin-Rezeptor-Liganden zur Behandlung von inflammatorischen und obstruktiven Atemwegserkrankungen, EP 1762568 und EP 1591443 als Inhibitoren der Plättchenaggregation, WO 07/002559 als Modulatoren der Aktivität von Nuklearrezeptoren, WO 07/020388 und WO 05/080343 als Cannabinoid-Rezeptor-Modulatoren unter anderem zur Behandlung von Fettsucht und psychiatrischen und neurologischen Störungen, WO 07/009701 und EP 1743637 zur Behandlung kardiovaskulärer Risikofaktoren, WO 2005/002576 als Inhibitoren verschiedener Kinasen und DE 10 2004 054 666 zur Bekämpfung von Schadpflanzen oder zur Wachstumsregulierung von Pflanzen.

Eine Aufgabe der vorliegenden Erfindung ist es daher, neue Verbindungen mit gleicher oder verbesserter antiviraler Wirkung zur Behandlung von viralen Infektionskrankheiten bei Menschen und Tieren zur Verfügung zu stellen, die die zuvor beschriebenen Nachteile nicht aufweisen.

Überraschenderweise wurde gefunden, dass die in der vorliegenden Erfindung beschriebenen substituierten Pyrazolamide antiviral wirksam sind.

Gegenstand der Erfindung ist die Verwendung der Verbindungen der Formel 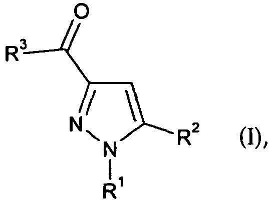 in welcher
- R¹: für Phenyl steht,
wobei Phenyl substituiert ist mit 1 bis 3 Substituenten, wobei die Substituenten unabhängig voneinander ausgewählt werden aus der Gruppe bestehend aus Halogen, Cyano, Nitro, Trifluormethyl, Trifluormethoxy, Trifluormethylthio, C₁-C₄-Alkyl und C₁-C₄-Alkoxy,
- R²: für Phenyl steht,
wobei Phenyl substituiert sein kann mit 1 bis 3 Substituenten, wobei die Substituenten unabhängig voneinander ausgewählt werden aus der Gruppe bestehend aus Halogen, Cyano, Nitro, Trifluormethyl, Trifluormethoxy, Trifluormethylthio, C₁-C₄-Alkyl und C₁-C₄-Alkoxy,
- R³: für einen über Stickstoff gebundenen 5- bis 8-gliedrigen Heterocyclus steht,
wobei der Heterocyclus substituiert sein kann mit 1 bis 3 Substituenten, wobei die Substituenten unabhängig voneinander ausgewählt werden aus der Gruppe bestehend aus Halogen, Hydroxy, Hydroxymethyl, Formyl, Amino, Oxo, Trifluormethyl, Trifluormethoxy, C₁-C₄-Alkyl, C₁-C₄-Alkoxy und C₁-C₄-Alkoxycarbonyl,
und ihre Salze, ihre Solvate und die Solvate ihrer Salze,
zur Herstellung eines Arzneimittels zur Behandlung und/oder Prophylaxe von retroviralen Erkrankungen.

Erfindungsgemäße Verbindungen sind die Verbindungen der Formel (I) und deren Salze, Solvate und Solvate der Salze, sowie die von Formel (I) umfassten, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb auch die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegenden Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:
Alkyl sowie die Alkylteile in Alkoxy und Alkoxycarbonyl stehen für geradkettiges oder verzweigtes Alkyl und umfassen, wenn nicht anders angegeben, C₁-C₆-Alkyl, insbesondere C₁-C₄-Alkyl, wie z.B. Methyl, Ethyl, Propyl, Isopropyl, Butyl, Isobutyl.
Alkoxy steht im Rahmen der Erfindung vorzugsweise für einen geradkettigen oder verzweigten Alkoxyrest insbesondere mit 1 bis 6, 1 bis 4 bzw. 1 bis 3 Kohlenstoffatomen. Bevorzugt ist ein geradkettiger oder verzweigter Alkoxyrest mit 1 bis 3 Kohlenstoffatomen. Beispielhaft und vorzugsweise seien genannt: Methoxy, Ethoxy, n-Propoxy, Isopropoxy, t-Butoxy, n-Pentoxy und n-Hexoxy.
Alkoxycarbonyl steht beispielhaft und vorzugsweise für Methoxycarbonyl, Ethoxycarbonyl, n-Propoxycarbonyl, Isopropoxycarbonyl, t-Butoxycarbonyl, n-Pentoxycarbonyl und n-Hexoxycarbonyl.
Heterocyclus steht für einen monocyclischen, heterocyclischen Rest mit 5 bis 8, vorzugsweise 5 bis 6 Ringatomen und bis zu 3, vorzugsweise bis zu 2 Heteroatomen und/oder Heterogruppen aus der Reihe N, O, S, SO, SO₂, wobei ein Stickstoffatom auch ein N-Oxid bilden kann. Der Heterocyclus kann gesättigt oder teilweise ungesättigt sein. Bevorzugt sind 5- bis 8-gliedrige, monocyclische gesättigte Heterocyclen mit bis zu zwei Heteroatomen aus der Reihe O, N und S, beispielhaft und vorzugsweise 1,4-Oxazepanyl, Oxetan-3-yl, Pyrrolidin-1-yl, Pyrrolidin-2-yl, Pyrrolidin-3-yl, Tetrahydrofuranyl, Tetrahydrothienyl, Pyranyl, 1,3-Thiazolidinyl, Piperidin-1-yl, Piperidin-2-yl, Piperidin-3-yl, Piperidin-4-yl, Thiopyranyl, Morpholin-2-yl, Morpholin-3-yl, Morpholin-4-yl, Thiomorpholin-2-yl, Thiomorpholin-3-yl, Thiomorpholin-4-yl, Perhydroazepinyl, Piperazin-1-yl, Piperazin-2-yl.
Halogen steht für Fluor, Chlor, Brom oder Jod, wobei Fluor und Chlor bevorzugt sind, wenn nichts anderes angegeben ist.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Die in den jeweiligen Kombinationen bzw. bevorzugten Kombinationen von Resten im einzelnen angegebenen Restedefinitionen werden unabhängig von den jeweilig angegebenen Kombinationen der Reste beliebig auch durch Restedefinitionen anderer Kombinationen ersetzt.

Gegenstand der Erfindung ist auch die Verwendung der Verbindungen der Formel (I), in welcher
- R¹: für Phenyl steht,
wobei Phenyl substituiert ist mit 1 bis 3 Substituenten, wobei die Substituenten unabhängig voneinander ausgewählt werden aus der Gruppe bestehend aus Halogen, Cyano, Nitro, Trifluormethyl, Trifluormethoxy, Trifluormethylthio, C₁-C₄-Alkyl und C₁-C₄-Alkoxy,
- R²: für Phenyl steht,
wobei Phenyl substituiert sein kann mit 1 bis 3 Substituenten, wobei die Substituenten unabhängig voneinander ausgewählt werden aus der Gruppe bestehend aus Halogen, Cyano, Nitro, Trifluormethyl, Trifluormethoxy, Trifluormethylthio, C₁-C₄-Alkyl und C₁-C₄-Alkoxy,
- R³: für Pyrrolidin-1-yl, Morpholin-4-yl, Thiomorpholin-4-yl, 1,3-Thiazolidin-3-yl, Piperidin-1-yl, Piperazin-1-yl oder 1,4-Oxazepan-4-yl steht,
wobei Pyrrolidin-1-yl, Morpholin-4-yl, Thiomorpholin-4-yl, 1,3-Thiazolidin-3-yl, Piperidin-1-yl, Piperazin-1-yl oder 1,4-Oxazepan-4-yl substituiert sein können mit 1 bis 3 Substituenten, wobei die Substituenten unabhängig voneinander ausgewählt werden aus der Gruppe bestehend aus Hydroxy, Hydroxy-methyl, Formyl, Amino, Oxo, C₁-C₄-Alkyl, C₁-C₄-Alkoxy und C₁-C₄-Alkoxy-carbonyl,
und ihre Salze, ihre Solvate und die Solvate ihrer Salze,
zur Herstellung eines Arzneimittels zur Behandlung und/oder Prophylaxe von retroviralen Erkrankungen.

Gegenstand der Erfindung ist auch die Verwendung der Verbindungen der Formel (I), in welcher
- R¹: für Phenyl steht,
wobei Phenyl substituiert ist mit 1 bis 3 Substituenten, wobei die Substituenten unabhängig voneinander ausgewählt werden aus der Gruppe bestehend aus Halogen, Cyano, Nitro, Trifluormethyl, Trifluormethoxy, Trifluormethylthio, C₁-C₄-Alkyl und C₁-C₄-Alkoxy,
- R²: für Phenyl steht,
wobei Phenyl substituiert sein kann mit 1 bis 3 Substituenten, wobei die Substituenten unabhängig voneinander ausgewählt werden aus der Gruppe bestehend aus Halogen, Cyano, Nitro, Trifluormethyl, Trifluormethoxy, Trifluormethylthio, C₁-C₄-Alkyl und C₁-C₄-Alkoxy,
und
- R³: für einen über Stickstoff gebundenen 5- bis 8-gliedrigen Heterocyclus steht,
wobei der Heterocyclus substituiert ist mit 1 bis 3 Substituenten, wobei die Substituenten unabhängig voneinander ausgewählt werden aus der Gruppe bestehend aus Halogen, Hydroxy, Hydroxymethyl, Formyl, Amino, Oxo, Trifluormethyl, Trifluormethoxy, C₁-C₄-Alkyl, C₁-C₄-Alkoxy und C₁-C₄-Alkoxy-carbonyl,
oder
- R¹: für Phenyl steht,
wobei Phenyl substituiert ist mit 1 bis 3 Substituenten, wobei die Substituenten unabhängig voneinander ausgewählt werden aus der Gruppe bestehend aus Halogen, Cyano, Nitro, Trifluormethyl, Trifluormethoxy, Trifluormethylthio, C₁-C₄-Alkyl und C₁-C₄-Alkoxy,
- R²: für Phenyl steht,
wobei Phenyl substituiert sein kann mit 1 bis 3 Substituenten, wobei die Substituenten unabhängig voneinander ausgewählt werden aus der Gruppe bestehend aus Trifluormethoxy und Trifluormethylthio,
und
- R³: für einen über Stickstoff gebundenen unsubstituierten 5- bis 8-gliedrigen Heterocyclus steht,
und ihre Salze, ihre Solvate und die Solvate ihrer Salze,
zur Herstellung eines Arzneimittels zur Behandlung und/oder Prophylaxe von retroviralen Erkrankungen.

Gegenstand der Erfindung sind weiterhin Verbindungen der Formel (I), in welcher
- R¹: für Phenyl steht,
wobei Phenyl substituiert ist mit 1 bis 3 Substituenten, wobei die Substituenten unabhängig voneinander ausgewählt werden aus der Gruppe bestehend aus Halogen, Cyano, Nitro, Trifluormethyl, Trifluormethoxy, Trifluormethylthio, C₁-C₄-Alkyl und C₁-C₄-Alkoxy,
- R²: für Phenyl steht,
wobei Phenyl substituiert sein kann mit 1 bis 3 Substituenten, wobei die Substituenten unabhängig voneinander ausgewählt werden aus der Gruppe bestehend aus Halogen, Cyano, Nitro, Trifluormethyl, Trifluormethoxy, Trifluormethylthio, C₁-C₄-Alkyl und C₁-C₄-Alkoxy,
und
- R³: für einen über Stickstoff gebundenen 5- bis 8-gliedrigen Heterocyclus steht,
wobei der Heterocyclus substituiert ist mit 1 bis 3 Substituenten, wobei die Substituenten unabhängig voneinander ausgewählt werden aus der Gruppe bestehend aus Halogen, Hydroxy, Hydroxymethyl, Formyl, Amino, Oxo, Trifluormethyl, Trifluormethoxy, C₁-C₄-Alkyl, C₁-C₄-Alkoxy und C₁-C₄-Alkoxy-carbonyl,
oder
- R¹: für Phenyl steht,
wobei Phenyl substituiert ist mit 1 bis 3 Substituenten, wobei die Substituenten unabhängig voneinander ausgewählt werden aus der Gruppe bestehend aus Halogen, Cyano, Nitro, Trifluormethyl, Trifluormethoxy, Trifluormethylthio, C₁-C₄-Alkyl und C₁-C₄-Alkoxy,
- R²: für Phenyl steht,
wobei Phenyl substituiert sein kann mit 1 bis 3 Substituenten, wobei die Substituenten unabhängig voneinander ausgewählt werden aus der Gruppe bestehend aus Trifluormethoxy und Trifluormethylthio,
und
- R³: für einen über Stickstoff gebundenen unsubstituierten 5- bis 8-gliedrigen Heterocyclus steht,
und ihre Salze, ihre Solvate und die Solvate ihrer Salze.

Gegenstand der Erfindung sind weiterhin Verbindungen der Formel (I), in welcher R² für Phenyl steht, wobei Phenyl substituiert ist mit einem Substituenten, wobei der Substituent in meta- oder para-Position zur Verknüpfungsstelle des Phenylrings an das Pyrazol steht.

Gegenstand der Erfindung sind weiterhin Verbindungen der Formel (I), in welcher R³ für Pyrrolidin-1-yl, Morpholin-4-yl, Thiomorpholin-4-yl, 1,3-Thiazolidin-3-yl, Piperidin-1-yl, Piperazin-1-yl oder 1,4-Oxazepan-4-yl steht, wobei Pyrrolidin-1-yl, Morpholin-4-yl, Thiomorpholin-4-yl, 1,3-Thiazolidin-3-yl, Piperidin-1-yl, Piperazin-1-yl oder 1,4-Oxazepan-4-yl substituiert sein können mit 1 bis 3 Substituenten, wobei die Substituenten unabhängig voneinander ausgewählt werden aus der Gruppe bestehend aus Hydroxy, Hydroxymethyl, Formyl, Amino, Oxo, C₁-C₄-Alkyl, C₁-C₄-Alkoxy und C₁-C₄-Alkoxycarbonyl.

Gegenstand der Erfindung sind weiterhin Verbindungen der Formel (I), in welcher R³ für Pyrrolidin-1-yl, Morpholin-4-yl, Thiomorpholin-4-yl, 1,3-Thiazolidin-3-yl, Piperidin-1-yl, Piperazin-1-yl oder 1,4-Oxazepan-4-yl steht.

Folgendes Verfahren wird zur Herstellung der Verbindungen der Formel (I) angewendet wobei Verbindungen der Formel 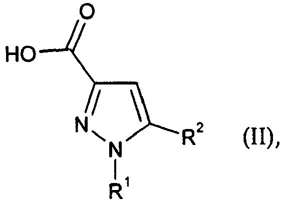 in welcher
R¹ und R² die oben angegebene Bedeutung aufweisen,
mit Verbindungen der Formel 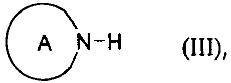 in welcher
A ein Heterocyclus wie zuvor für R³ definiert ist,
umgesetzt werden.

Die Umsetzung erfolgt im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoffe wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin.

Als Dehydratisierungsreagenzien eignen sich hierbei beispielsweise Carbodiimide wie z.B. *N*,*N'*-Diethyl-, *N,N,'*-Dipropyl-, *N,N'*-Diisopropyl-, *N,N'*-Dicyclohexylcarbodiimid, *N*-(3-Dimethylaminoisopropyl)-*N'*-ethylcarbodiimid-hydrochlorid (EDC), *N*-Cyclo-hexylcarbodiimid-*N'*-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert*-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid, oder *O*-(Benzotriazol-1-yl)-*N,N,N',N'*-tetramethyluroniumhexafluorophosphat (HBTU), 2-(2-Oxo-1-(2H)-pyridyl)-1,1,3,3-tetramethyluroniumtetrafluoroborat (TPTU) oder *O*-(7-Azabenzotriazol-1-yl)-*N*,*N*,*N'*,*N'-*tetramethyluroniumhexafluorophosphat (HATU), oder 1-Hydroxybenzotriazol (HOBt), oder Benzotriazol-1-yloxytris(dimethylamino)phosphoniumhexafluorophosphat (BOP), oder Benzotriazol-1-yloxytris(pyrrolidino)phosphoniumhexafluorophosphat (PyBOP), oder *N*-Hydroxysuccinimid, oder Mischungen aus diesen, mit Basen.

Vorzugsweise wird die Kondensation mit TBTU oder mit EDC in Gegenwart von HOBt durchgeführt.

In einem alternativen Verfahren können die Verbindungen der Formel (II) zunächst mit Thionylchlorid und in der zweiten Stufe mit Verbindungen der Formel (III) in Gegenwart einer Base, wie z. B. Triethylamin, umgesetzt werden.

Die nach den oben angegebenen Verfahren hergestellten Verbindungen der Formel (I) tragen gegebenenfalls Schutzgruppen, die nach dem Fachmann bekannten Bedingungen abgespalten werden können, um weitere Verbindungen der Formel (I) zu erhalten.

Die Verbindungen der Formel (III) sind bekannt oder lassen sich nach bekannten Verfahren aus den entsprechenden Edukten synthetisieren.

Die Verbindungen der Formel (II) sind bekannt oder und können hergestellt werden, indem in Verbindungen der Formel 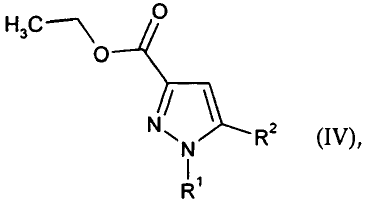 in welcher
R¹ und R² die oben angegebene Bedeutung aufweisen,
der Ester mit einer Base verseift wird.

Die Verseifung des Esters mit einer Base erfolgt im allgemeinen in inerten Lösungsmitteln, bevorzugt in einem Temperaturbereich von Raumtemperatur bis zum Rückfluss des Lösungsmittels bei Normaldruck.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, bevorzugt ist Lithium- oder Natriumhydroxid.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-*tert*-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Dimethylsulfoxid, Acetonitril oder Pyridin, oder Wasser, oder Gemische von Lösungsmitteln, als Lösungsmittel sind bevorzugt Tetrahydrofuran und/oder Methanol. Bevorzugt ist Kaliumhydroxid in Methanol.

Die Verbindungen der Formel (IV) sind bekannt oder und können hergestellt werden, indem in der ersten Stufe Verbindungen der Formel 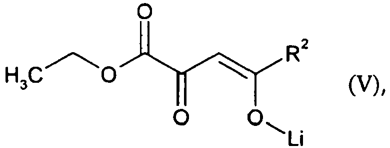 in welcher
R² die oben angegebene Bedeutung aufweist,
mit Verbindungen der Formel

R¹-NH-NH₂ (VI),

in welcher
R¹ die oben angegebene Bedeutung aufweist,
umgesetzt werden und in der zweiten Stufe in Essigsäure erhitzt wird.

Die Umsetzung der ersten Stufe erfolgt im allgemeinen in inerten Lösungsmitteln, bevorzugt in einem Temperaturbereich von Raumtemperatur bis zum Rückfluss des Lösungsmittels bei Normaldruck.

Inerte Lösungsmittel sind beispielsweise Alkohole wie Methanol, Ethanol, n-Propanol, iso-Propanol, n-Butanol, tert-Butanol oder 2-Methoxyethanol, bevorzugt ist Ethanol.

Die Umsetzung der zweiten Stufe in Essigsäure erfolgt im allgemeinen in einem Temperaturbereich von Raumtemperatur bis zum Rückfluss der Essigsäure bei Normaldruck. Die Reaktion kann auch in Methanol, Ethanol oder Dioxan in einem Temperaturbereich von Raumtemperatur bis zum Rückfluss der Lösungsmittel durchgeführt werden. Geeignet sind Mischungen von Methanol, Ethanol oder Dioxan mit Essigsäure im Volumenverhältnis von 0.5/99.5 bis 99.5/0.5. Auch können Mischungen von Methanol, Ethanol, Dioxan oder Essigsäure mit anderen Säuren wie z.B. Salzsäure, Methansulfonsäure, p-Toluolsulfonsäure, Camphersulfonsäure oder Trifluoressigsäure unter den genannten Bedingungen eingesetzt werden. Bevorzugt wird die Umsetzung in Essigsäure unter Rückfluss durchgeführt.

Die Verbindungen der Formeln (V) und (VI) sind bekannt oder lassen sich nach bekannten Verfahren aus den entsprechenden Edukten synthetisieren.

Die Herstellung der erfindungsgemäßen Verbindungen kann durch das folgende Syntheseschema verdeutlicht werden.

### Syntheseschema:

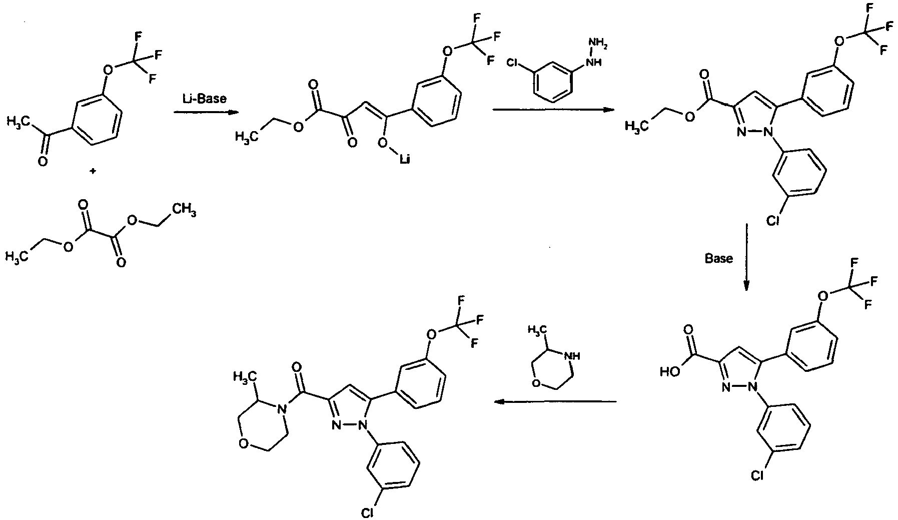

Die erfindungsgemäßen Verbindungen zeigen ein nicht vorhersehbares, wertvolles pharmakologisches Wirkspektrum.

Sie eignen sich daher zur Verwendung als Arzneimittel zur Behandlung und/oder Prophylaxe von Krankheiten bei Menschen und Tieren.

Die Verbindungen der vorliegenden Erfindung zeichnen sich insbesondere durch ein vorteilhaftes anti-retrovirales Wirkspektrum aus.

Weiterer Gegenstand der vorliegenden Erfindung ist der Einsatz der erfindungsgemäßen Verbindungen zur Behandlung und/oder Prophylaxe von Erkrankungen, die durch Retroviren hervorgerufen werden, insbesondere von HI-Viren.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer therapeutisch wirksamen Menge der erfindungsgemäßen Verbindungen.

Als Indikationsgebiete in der Humanmedizin können beispielsweise genannt werden:
1.) Die Behandlung und Prophylaxe von menschlichen Retrovirusinfektionen
2.) Die Behandlung und Prophylaxe von durch HIV I (Virus der humanen Immundefizienz; früher HTLV III/LAV genannt) und HIV II verursachten Infektionen und Erkrankungen (AIDS) und den damit assoziierten Stadien wie ARC (AIDS related complex) und LAS (Lymphadenopathie-Syndrom) sowie der durch dieses Virus verursachten Immunschwäche und Enzephalopathie.
3.) Die Behandlung von HIV-Infektionen hervorgerufen durch Einfach-, Mehrfach- oder Multi-resistente HIV Viren.

Der Begriff resistente HI-Viren bedeutet im Rahmen der Erfindung, z.B. Viren mit Resistenzen gegen nukleosidische Inhibitoren (RTI), nicht nukleosidische Inhibitoren (NNRTI) oder Proteaseinhibitoren (PI) oder Viren mit Resistenzen gegen andere Wirkprinzipien z.B. T20 (Fusionsinhibitoren).
4.) Die Behandlung oder die Prophylaxe des AIDS-carrier Zustandes (AIDS-Überträger-Zustand).
5.) Die Behandlung oder die Prophylaxe einer HTLV-I oder HTLV-II Infektion.

Als Indikationen in der Tiermedizin können beispielsweise angeführt werden:
Infektionen mit
   a) Maedivisna (bei Schafen und Ziegen)
   b) progressivem Pneumonievirus (PPV) (bei Schafen und Ziegen)
   c) caprine arthritis encephalitis Virus (bei Schafen und Ziegen)
   d) Zwoegerziekte Virus (bei Schafen)
   e) infektiösem Virus der Anämie (des Pferdes)
   f) Infektionen verursacht durch das Katzenleukämievirus
   g) Infektionen verursacht durch das Virus der Katzen-Immundefizienz (FIV)
   h) Infektionen verursacht durch das Virus der Affen-Immundefizienz (SIV)

Bevorzugt werden aus dem Indikationsgebiet in der Humanmedizin die oben aufgeführten Punke 2, 3 und 4.

Insbesondere geeignet sind die Substanzen zur Bekämpfung von HI Viren, die Resistenzen gegen bekannte nichtnukleosidische Inhibitoren der reversen Transkripatse wie z.B. Efavirenz oder Nevirapin zeigen.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen.

Die erfindungsgemäßen Verbindungen können, insbesondere in den oben aufgeführten Punkten 2, 3 und 4, auch vorteilhaft als Bestandteile einer Kombinationstherapie mit einer oder mehreren anderen, in diesen Anwendungsbereichen aktiven Verbindungen eingesetzt werden. Beispielhaft können diese Verbindungen in Kombination mit wirksamen Dosen von antiviral wirksamen Substanzen, die auf den unten aufgeführten Wirkprinzipien beruhen, eingesetzt werden:
Inhibitoren der HIV Protease; beispielhaft seien genannt: Saquinavir, Indinavir, Ritonavir, Nelfinavir, Amprenavir, Lopinavir, Atazanavir, Fosamprenavir, Tipranavir, Darunavir;
Nukleosidische, nukleotidische und nicht-nukleosidische Inhibitoren der HIV Reversen Transkriptase; beispielhaft seien genannt: Zidovudin, Lamivudin, Didanosin, Zalcitabin, Stavudin, Lamivudin, Abacavir, Tenofovir, Adefovir, Emtricitabin, Amdoxovir, Apricitabin, Racivir, Nevirapin, Delavirdin, Efavirenz, Etravirin, Rilpivirin, UK-453,061;
Inhibitoren der HIV Integrase; beispielhaft seien genannt: Raltegravir, Elvitegravir;
Inhibitoren der HIV Fusion; beispielhaft sei genannt: Enfuvirtid;
Inhibitioren der CXCR4/CCR5/gp120 Wechselwirkung; beispielhaft seien genannt: Maraviroc, Vicriviroc, INCB009471, AMD-070;
Inhibitioren der Polyproteinreifung; beispielhaft sei genannt: Bevirimat.

Diese Auswahl soll zur Verdeutlichung der Kombinationsmöglichkeiten, nicht jedoch zur Einschränkung auf die hier aufgeführten Beispiele dienen; prinzipiell ist jede Kombination der erfindungsgemäßen Verbindungen mit antiviral wirksamen Substanzen als im Rahmen der Erfindung zu betrachten.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich sowohl in der Human- als auch in der Veterinärmedizin als vorteilhaft erwiesen, den oder die erfindungsgemäßen Wirkstoffe in Gesamtmengen von 0.1 bis 200 mg/kg, vorzugsweise 1 bis 100 mg/kg Körpergewicht je 24 Stunden, gegebenenfalls in Form mehrerer Einzelgaben, zur Erzielung des gewünschten Ergebnisses zu verabreichen. Eine Einzelgabe enthält den oder die Wirkstoffe vorzugsweise in Mengen von 1 bis 80 mg/kg, insbesondere 1 bis 30 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10 % w/v": 100 ml Lösung oder Suspension enthalten 10 g Substanz.

### A) Beispiele

### Abkürzungen:

- aq.: wässrig, wässrige Lösung
- DCI: direkte chemische Ionisation (bei MS)
- DMA: *N*,*N*-Dimethylacetamid
- DMF: *N*,*N*-Dimethylformamid
- DMSO: Dimethylsulfoxid
- d. Th.: der Theorie (bei Ausbeute)
- EDC: *N*'-(3-Dimethylaminopropyl)-N-ethylcarbodiimid x HCl
- eq.: Äquivalent(e)
- ESI: Elektrospray-Ionisation (bei MS)
- h: Stunde(n)
- HATU: *O*-(7-Azabenzotriazol-1-yl)-*N*,*N*,*N'*,*N'*-tetramethyluronium-hexafluorophosphat
- HPLC: Hochdruck-, Hochleistungsflüssigchromatographie
- konz.: konzentriert
- LC-MS: Flüssigchromatographie-gekoppelte Massenspektroskopie
- min: Minute(n)
- MS: Massenspektroskopie
- NMR: Kernresonanzspektroskopie
- PyBOP: Benzotriazol-1-yloxytris(pyrrolidino)phosphonium-hexafluorophosphat
- Rₜ: Retentionszeit (bei HPLC)
- RT: Raumtemperatur
- TBTU: *O*-(Benzotriazol-1-yl)-*N,N,N',N'*-tetramethyluronium-tetrafluoroborat
- TFA: Trifluoressigsäure
- THF: Tetrahydrofuran
- TMOF: Trimethylorthoformiat

### HPLC-Methoden:

**Methode 1:** Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Temperatur: 30°C; UV-Detektion: 210 nm.
**Methode 2:** Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 9 min 90% B → 9.2 min 2% B → 10 min 2% B; Fluss: 0.75 ml/min; Temperatur: 30°C; UV-Detektion: 210 nm.
**Methode 3:** Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 15 min 90% B → 15.2 min 2% B → 16 min 2% B; Fluss: 0.75 ml/min; Temperatur: 30°C; UV-Detektion: 210 nm.

### LC/MS-Methoden:

**Methode 1:** Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.
**Methode 2:** Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.
**Methode 3:** Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.
**Methode 4:** Instrument MS: Micromass TOF (LCT); Instrument HPLC: 2-Säulen-Schaltung, Waters 2690; Säule: YMC-ODS-AQ, 50 mm x 4.6 mm, 3.0 µm; Eluent A: Wasser + 0.1% Ameisensäure, Eluent B: Acetonitril + 0.1% Ameisensäure; Gradient: 0.0 min 100% A → 0.2 min 95% A → 1.8 min 25% A → 1.9 min 10% A → 2.0 min 5% A → 3.2 min 5% A; Ofen: 40°C; Fluss: 3.0 ml/min; UV-Detektion: 210 nm.

### Ausgangsverbindungen

### Beispiel 1A

### Lithium (1Z)-4-Ethoxy-3,4-dioxo-1-[3-(trifluormethyl)phenyl]but-1-en-1-olat

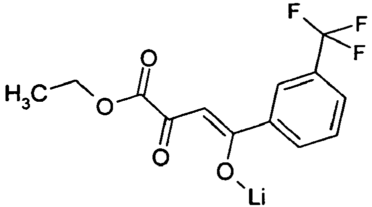

Eine Lösung aus 5.7 ml (5.7 mmol) Lithiumhexamethyldisilazid (Lösung in Hexan) in Diethylether wird bei -78°C vorgelegt. 1 g (5.31 mmol) 3-(Trifluormethyl)acetophenon wird in 6 ml Diethylether gelöst und zugetropft. Nach 45 Minuten bei -78°C werden 0.79 ml (5.85 mmol) Oxalsäurediethylester zugetropft und 12 Stunden bei RT gerührt. Das Reaktionsgemisch wird auf die Hälfte eingedampft und der erhaltene Feststoff abgesaugt. Die Kristalle werden mir Diethylether gewaschen und im Vakuum getrocknet. Es werden 1.26 g (4.3 mmol, 81% Ausbeute d. Th.) als Produkt erhalten. Das erhaltene Produkt wird direkt in die nächste Stufe eingesetzt.

### Beispiel 2A

### Lithium (1Z)-4-Ethoxy-3,4-dioxo-1-{3-[(trifluormethyl)thio]phenyl}but-1-en-1-olat

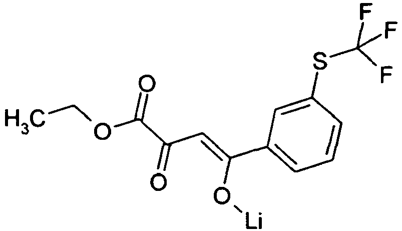

Ausgehend von 1 g (4.54 mmol) 3-(Trifluormetylthio)acetophenon und 0.68 ml (5 mmol) Oxalsäurediethylester werden nach dem in Beispiel 1A beschriebenen Verfahren 1.22 g (3.7 mmol, 82% Ausbeute d. Th.) als Produkt erhalten. Das erhaltene Produkt wird direkt in die nächste Stufe eingesetzt.

### Beispiel 3A

### Lithium (1Z)-4-Ethoxy-3,4-dioxo-1-[3-(trifluormethoxy)phenyl]but-1-en-1-olat

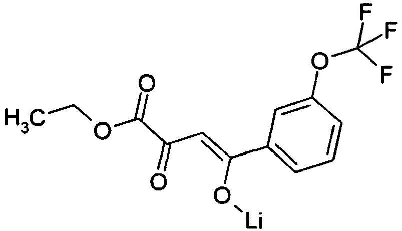

Ausgehend von 5 g (24.5 mmol) 1-[3-(Trifluormethoxy)phenyl]ethan-1-on und 3.66 ml (26.9 mmol) Oxalsäurediethylester werden nach dem in Beispiel 1A beschriebenen Verfahren 4.53 g (14.6 mmol, 60% Ausbeute d. Th.) als Produkt erhalten. Das erhaltene Produkt wird direkt in die nächste Stufe eingesetzt.

### Beispiel 4A

### 1-(3-Chlorphenyl)-5-[3-(trifluormethyl)phenyl]-1H-pyrazol-3-carbonsäureethylester

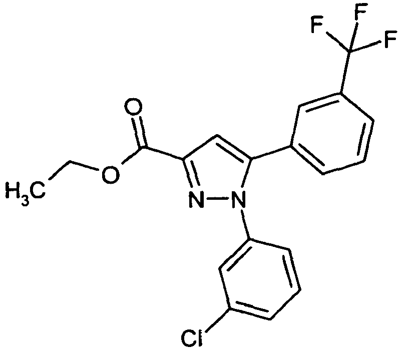

631.5 mg (2.15 mmol) Lithium (1Z)-4-Ethoxy-3,4-dioxo-1-[3-(trifluormethyl)-phenyl]but-1-en-1-olat aus Beispiel 1A wird in 15 ml Ethanol suspendiert, mit 522.7 mg (2.92 mmol) 3-Chlorphenylhydrazinhydrochlorid versetzt und 48 Stunden bei Raumtemperatur gerührt. Das Reaktionsgemisch wird auf ca. 5 ml eingedampft und der ausgefallene Feststoff wird abgesaugt. Nach Trocknen der Kristalle werden diese in 15 ml Essigsäure aufgenommen und 12 Stunden bei Rückfluss gerührt. Der Ansatz wird auf Essigsäureethylester gegeben, mit Wasser, ges. NatriumhydrogencarbonatLösung und mit Natriumchlorid-Lösung gewaschen. Die organische Phase wird über Natriumsulfat getrocknet und einrotiert. Der erhaltene Rückstand wird über eine Kieselgelflash (Laufmittel: Cyclohexan/ Essigsäureethylester 3:1) gereinigt und anschließend aus Diethylether/ Pentan kristallisiert. Es werden 528 mg (1.5 mmol, 62% Ausbeute d. Th.) Produkt erhalten.
Schmelzpunkt: 114°C
HPLC (Methode 1): Rₜ = 5.39 min
MS (ESIpos): m/z = 395 (M+H)⁺
¹H-NMR (400 MHz, DMSO-d₆): δ = 7.78 (d, 1H), 7.68-7.54 (m, 5H), 7.5 (t, 1H), 7.35 (s, 1H), 7.29 (d, 1H), 4.36 (q, 2H), 1.33 (t, 3H).

### Beispiel 5A

### 1-(3-Chlorphenyl)-5-{3-[(trifluormethyl)thio]phenyl}-1H-pyrazol-3-carbonsäureethylester

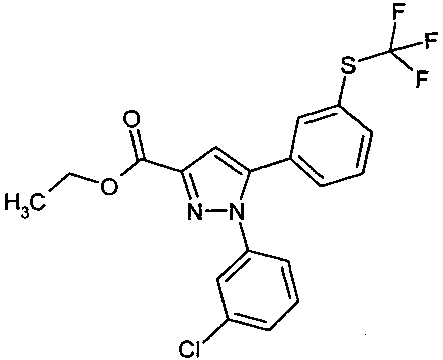

Ausgehend von 1.22 g (3.74 mmol) Lithium (1Z)-4-Ethoxy-3,4-dioxo-1-{3-[(trifluormethyl)thio]phenyl}but-1-en-1-olat aus Beispiel 2A und 568.35 mg (3.63 mmol) (3-Chlorbenzyl)hydrazin werden nach dem in Beispiel 4A beschriebenen Verfahren 845.1 mg (2.1 mmol, 77% Ausbeute d. Th.) Produkt erhalten.
Schmelzpunkt: 95°C
HPLC (Methode 1): Rₜ = 5.58 min
MS (ESIpos): m/z = 427 (M+H)⁺
¹H-NMR (300 MHz, DMSO-d₆): δ = 7.75 (d, 1H), 7.7-7.42 (m, 6H), 7.33-7.22 (m, 2H), 4.36 (q, 2H), 1.32 (t, 3H).

### Beispiel 6A

### 1-(3-Chlorphenyl)-5-[3-(trifluormethoxy)phenyl]-1H-pyrazol-3-carbonsäureethylester

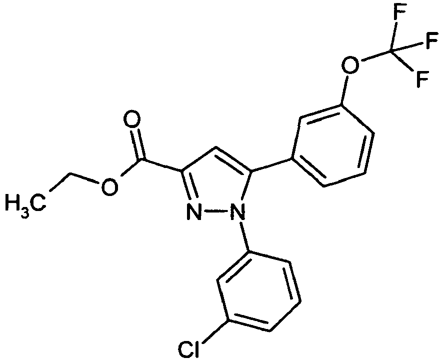

Ausgehend von 4.53 g (14.6 mmol) Lithium (1Z)-4-Ethoxy-3,4-dioxo-1-[3-(trifluormethoxy)phenyl]but-1-en-1-olat aus Beispiel 3A und 3.56 g (19.8 mmol) 3-Chlorphenylhydrazin-hydrochlorid werden nach dem in Beispiel 4A beschriebenen Verfahren 1.98 g (4.8 mmol, 33% Ausbeute d. Th.) Produkt erhalten.
Schmelzpunkt: 87°C
HPLC (Methode 1): Rₜ = 5.38 min
MS (ESIpos): m/z = 411 (M+H)⁺
¹H-NMR (400 MHz, DMSO-d₆): δ = 7.6-7.45 (m, 4H), 7.4 (d, 2H), 7.34-7.25 (m, 2H), 7.2 (s, 1H), 4.35 (q, 2H), 1.32 (t, 3H).

### Beispiel 7A

### 1-(3-Chlorphenyl)-5-[3-(trifluormethyl)phenyl]-1H-pyrazol-3-carbonsäure

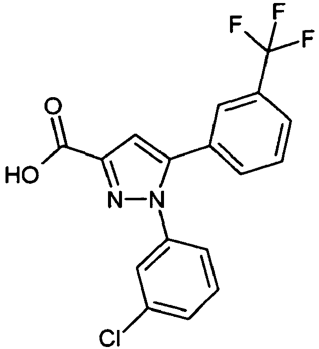

Eine Lösung aus 6 g (15.2 mmol) 1-(3-Chlorphenyl)-5-[3-(trifluormethyl)phenyl]-1H-pyrazol-3-carbonsäureethylester aus Beispiel 4A in 80 ml Methanol wird mit 8.5 g (152 mmol) Kaliumhydroxid versetzt und 30 Minuten bei Rückfluss gerührt. Das Reaktionsgemisch wird mit Wasser verdünnt und mit 1 molarer Salzsäure sauer gestellt. Es wird mit Essigsäureethylester ausgeschüttelt. Die organische Phase wird mit Natriumchlorid-Lösung gewaschen, über Natriumsulfat getrocknet und einrotiert. Der erhaltene Rückstand wird aus Diethylether/ Pentan kristallisiert. Die Kristalle werden abgesaugt, mit wenig Pentan gewaschen und getrocknet. Es werden 5.2 g (14.2 mmol, 93% Ausbeute d. Th.) Produkt erhalten.
HPLC (Methode 2): Rₜ = 4.57 min
MS (ESIpos): m/z = 367 (M+H)⁺
¹H-NMR (300 MHz, DMSO-d₆): δ = 13.15 (s, 1H), 7.78 (d, 1H), 7.68-7.43 (m, 6H), 7.3-7.23 (m, 2H).

### Beispiel 8A

### 1-(3-Chlorphenyl)-5-{3-[(trifluormethyl)thio]phenyl}-1H-pyrazol-3-carbonsäure

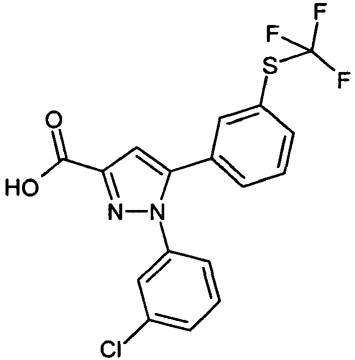

Ausgehend von 850 mg (1.99 mmol) 1-(3-Chlorphenyl)-5-{3-[(trifluormethyl)-thio]phenyl}-1H-pyrazol-3-carbonsäureethylester aus Beispiel 5A und 1.12 g (19.9 mmol) Kaliumhydroxid werden nach dem in Beispiel 7A beschriebenen Verfahren 691.5 mg (1.7 mmol, 87% Ausbeute d. Th.) als Kristalle erhalten.
Schmelzpunkt: 148°C
HPLC (Methode 1): Rₜ = 4.91 min
MS (ESIpos): m/z = 399 (M+H)⁺
¹H-NMR (300 MHz, DMSO-d₆): δ = 13.15 (s, 1H), 7.74 (d, 1H), 7.7-7.42 (m, 6H), 7.3 (d, 1H), 7.2 (s, 1H).

### Beispiel 9A

### 1-(3-Chlorphenyl)-5-[3-(trifluormethoxy)phenyl]-1H-pyrazol-3-carbonsäure

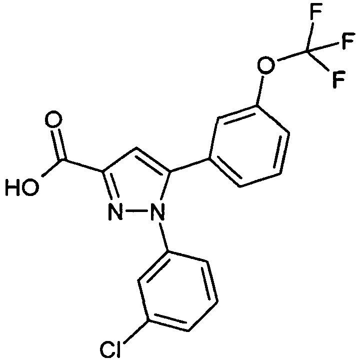

Ausgehend von 1.9 g (4.63 mmol) 1-(3-Chlorphenyl)-5-[3-(trifluormethoxy)phenyl]-1H-pyrazol-3-carbonsäureethylester aus Beispiel 6A und 2.59 g (46.25 mmol) Kaliumhydroxid werden nach dem in Beispiel 7A beschriebenen Verfahren 1.68 g (4.4 mmol, 95% Ausbeute d. Th.) als Kristalle erhalten.
HPLC (Methode 2): Rₜ = 4.76 min
¹H-NMR (400 MHz, DMSO-d₆): δ = 7.6-7.45 (m, 4H), 7.44-7.38 (m, 2H), 7.28 (d, 1H), 7.2 (d, 2H).

### Beispiel 10A

### Lithium (1Z)-1-(3-Cyanophenyl)-4-ethoxy-3,4-dioxobut-1-en-1-olat

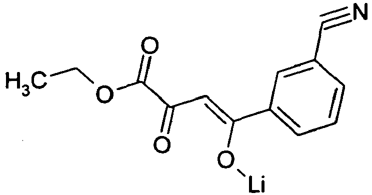

Ausgehend von 5 g (34.4 mmol) 3-Acetylbenzonitril und 5.15 ml (37.9 mmol) Oxalsäure-diethylester werden nach dem in Beispiel 1A beschriebenen Verfahren 5.49 g (21.9 mmol, 63% Ausbeute d. Th.) als Produkt erhalten. Das erhaltene Produkt wird direkt in die nächste Stufe eingesetzt.

### Beispiel 11A

### 4-(2,4-Dichlorphenyl)-2,4-dioxobutansäureethylester

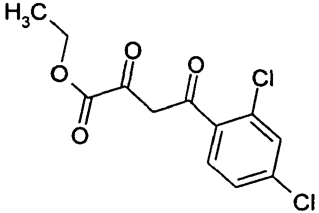

Die Herstellung erfolgt nach Bioorganic & Medicinal Chemistry Letters 12 (16), 2133 (2002).

### Beispiel 12A

### 4-(3,4-Dimethoxyphenyl)-2,4-dioxobutansäureethylester

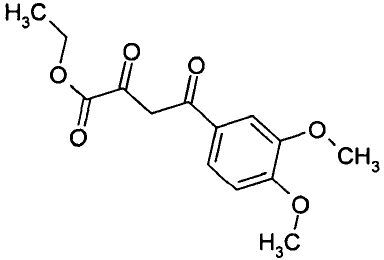

Die Herstellung erfolgt nach Bioorganic & Medicinal Chemistry Letters 12 (16), 2133 (2002).

### Beispiels 13A

### 2,4-Dioxo-4-phenylbutansäureethylester

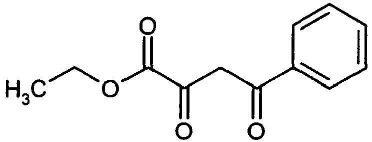

Die Herstellung erfolgt nach A.Roy and S.Batra, Synthesis (15), 2325 (2003).

### Beispiel 14A

### 3-(3-Nitrophenyl)-3-oxopropansäureethylester

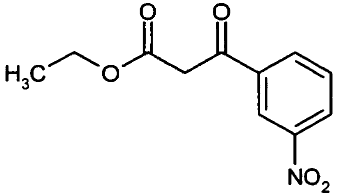

Die Herstellung erfolgt nach Tetrahedron 60(31), 6479 (2004).

### Beispiel 15A

### 1-(4-Methylphenyl)-5-[3-(trifluormethyl)phenyl]-1H-pyrazol-3-carbonsäureethylester

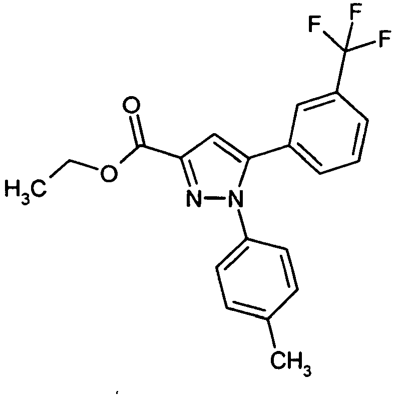

Ausgehend von 631.5 mg (2.15 mmol) Lithium (1Z)-4-Ethoxy-3,4-dioxo-1-[3-(trifluormethyl)phenyl]but-1-en-1-olat aus Beispiel 1A und 463.2 mg (2.92 mmol) 4-Tolylhydrazinhydrochlorid werden nach dem in Beispiel 4A beschriebenen Verfahren 535.8 mg (1.4 mmol, 67% Ausbeute d. Th.) Produkt erhalten.
Schmelzpunkt: 102°C
HPLC (Methode 1): Rₜ = 5.36 min
MS (ESIpos): m/z = 375 (M+H)⁺
¹H-NMR (400 MHz, DMSO-d₆): δ = 7.73 (d, 1H), 7.64-7.5 (m, 3H), 7.32-7.2 (m, 5H), 4.33 (q, 2H), 2.36 (s, 3H), 1.32 (t, 3H).

### Beispiel 16A

### 1-(3-Chlorphenyl)-5-(3-cyanophenyl)-1H-pyrazol-3-carbonsäureethylester

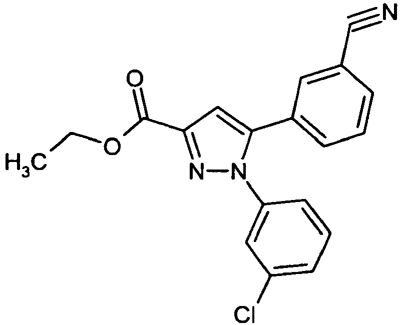

Ausgehend von 10 g (39.81 mmol) Lithium (1Z)-1-(3-Cyanophenyl)-4-ethoxy-3,4-dioxobut-1-en-1-olat aus Beispiel 10A und 9.7 g (54.15 mmol) 3-Chlorphenylhydrazinhydrochlorid werden nach dem in Beispiel 4A beschriebenen Verfahren 4.97 g (14 mmol, 35% Ausbeute d. Th.) Produkt erhalten.
Schmelzpunkt: 101°C
LC-MS (Methode 1): Rₜ = 2.45 min
MS (ESIpos): m/z = 352 (M+H)⁺
¹H-NMR (400 MHz, DMSO-d₆): δ = 7.38 (d, 2H), 7.6-7.43 (m, 5H), 7.3 (s, 1H), 7.28 (d, 1H), 4.35 (q, 2H), 1.32 (t, 3H).

### Beispiel 17A

### 1-(3-Chlorphenyl)-5-(3,4-dimethoxyphenyl)-1H-pyrazol-3-carbonsäureethylester

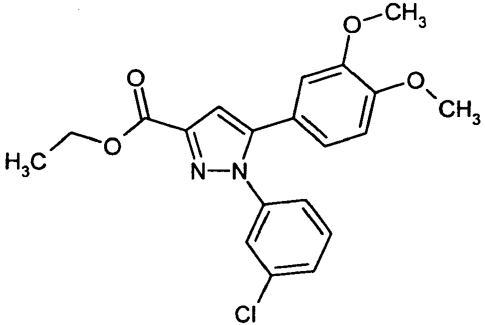

Ausgehend von 1 g (3.57 mmol) 4-(3,4-Dimethoxyphenyl)-2,4-dioxobutansäure-ethylester aus Beispiel 12A und 868.8 mg (4.85 mmol) 3-Chlorphenylhydrazinhydrochlorid werden nach dem in Beispiel 4A beschriebenen Verfahren und nach Reinigung über präparative HPLC 1.03 g (2.7 mmol, 74% Ausbeute d. Th.) Produkt erhalten.
Schmelzpunkt: 99°C
HPLC (Methode 2): Rₜ = 4.81 min
MS (ESIpos): m/z = 387 (M+H)⁺
¹H-NMR (400 MHz, DMSO-d₆): δ = 7.57-7.44 (m, 3H), 7.27 (d, 1H), 7.13 (s, 1H), 6.95 (d, 1H), 6.88 (s, 1H), 6.77 (d, 1H), 4.34 (q, 2H), 3.75 (s, 3H), 3.62 (s, 3H), 1.32 (t, 3H).

### Beispiel 18A

### 1-(3-Chlorphenyl)-5-(2,4-dichlorphenyl)-1H-pyrazol-3-carbonsäureethylester

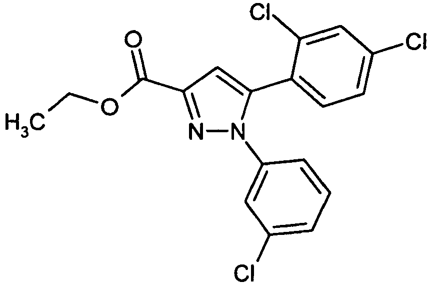

Ausgehend von 1 g (3.46 mmol) 4-(2,4-Dichlorphenyl)-2,4-dioxobutansäureethylester aus Beispiel 11A und 842.25 mg (4.7 mmol) 3-Chlorphenylhydrazinhydrochlorid werden nach dem in Beispiel 4A beschriebenen Verfahren und nach Reinigung über präparative HPLC 297.5 mg (0.75 mmol, 22% Ausbeute d. Th.) Produkt erhalten.
Schmelzpunkt: 87°C
HPLC (Methode 2): Rₜ = 5.49 min
¹H-NMR (400 MHz, DMSO-d₆): δ = 7.75 (s, 1H), 7.63-7.54 (m, 2H), 7.52-7.39 (m, 3H), 7.2-7.1 (m, 2H), 4.35 (q, 2H), 1.33 (t, 3H).

### Beispiel 19A

### 1-(3-Chlorphenyl)-5-phenyl-1H-pyrazol-3-carbonsäureethylester

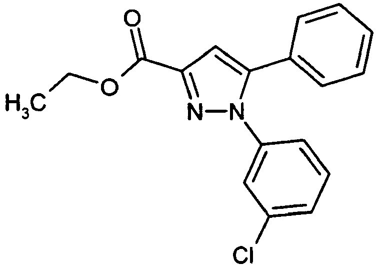

Die Herstellung erfolgt nach Il Farmaco 59(11), 849 (2004).

### Beispiel 20A

### 1-(3-Chlorphenyl)-5-(3-nitrophenyl)-1 H-pyrazol-3-carbonsäureethylester

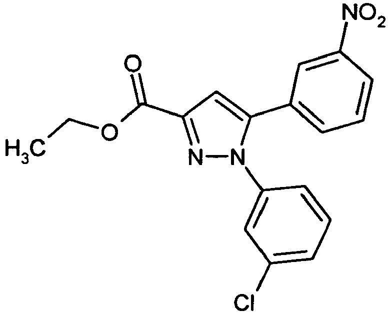

Ausgehend von 1 g (3.77 mmol) 3-(3-Nitrophenyl)-3-oxopropansäureethylester aus Beispiel 14A und 918.13 mg (5.13 mmol) 3-Chlorphenylhydrazinhydrochlorid werden nach dem in Beispiel 4A beschriebenen Verfahren und nach Reinigung über präparative HPLC 874.5 mg (2.4 mmol, 62% Ausbeute d. Th.) Produkt erhalten.
Schmelzpunkt: 116°C
HPLC (Methode 2): Rₜ = 5.33 min
MS (ESIpos): m/z = 372 (M+H)⁺
¹H-NMR (400 MHz, DMSO-d₆): δ = 8.39 (d, 1H), 8.3 (s, 1H), 7.89-7.78 (m, 2H), 7.78-7.68 (m, 2H), 7.63 (t, 1H), 7.53 (s, 1H), 7.46 (d, 1H).

### Beispiel 21A

### 1-(3-Methoxyphenyl)-5-[3-(trifluormethyl)phenyl]-1H-pyrazol-3-carbonsäureethylester

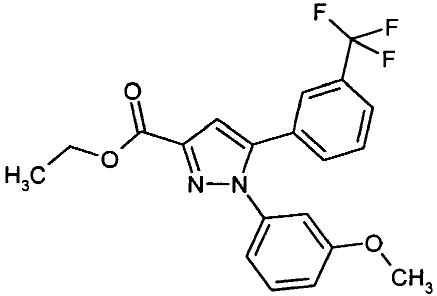

Ausgehend von 1.5 g (5.2 mmol) Lithium (1Z)-4-Ethoxy-3,4-dioxo-1-[3-(trifluormethyl)phenyl]-but-1-en-1-olat aus Beispiel 1A und 1 g (5.73 mmol) 3-Methoxyphenylhydrazinhydrochlorid werden nach dem in Beispiel 4A beschriebenen Verfahren und nach Reinigung über Kieselgel (Flashchromatographie) 1.73 g (4.4 mmol, 85% Ausbeute d. Th.) Produkt erhalten.
LC-MS (Methode 2): Rₜ = 2.95 min
MS (ESIpos): m/z = 391 (M+H)⁺
¹H-NMR (400 MHz, CDCl₃): δ = 7.59 (d, 1H), 7.53 (s, 1H), 7.43 (t, 1H), 7.37 (d, 1H), 7.28-7.14 (m, 2H), 6.93 (s, 2H), 6.8 (d, 1H), 4.46 (q, 2H), 3.75 (s, 3H), 1.43 (t, 3H).

### Beispiel 22A

### 1-(4-Methylphenyl)-5-[3-(trifluormethyl)phenyl]-1H-pyrazol-3-carbonsäure

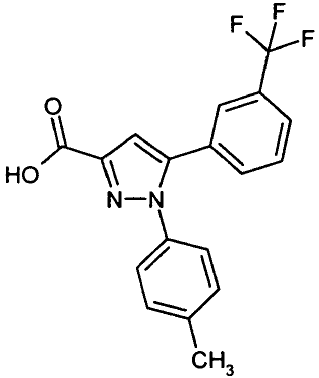

Ausgehend von 450 mg (1.2 mmol) 1-(4-Methylphenyl)-5-[3-(trifluormethyl)phenyl]-1H-pyrazol-3-carbonsäureethylester aus Beispiel 15A und 135 mg (2.4 mmol) Kaliumhydroxid werden nach dem in Beispiel 7A beschriebenen Verfahren 334.7 mg (0.97 mmol, 80% Ausbeute d. Th.) als Kristalle erhalten.
Schmelzpunkt: 151°C
HPLC (Methode 1): Rₜ = 4.69 min
MS (ESIpos): m/z = 347 (M+H)⁺
¹H-NMR (300 MHz, DMSO-d₆): δ = 13.08 (s, 1H), 7.72 (d, 1H), 7.63-7.5 (m, 3H), 7.31-7.18 (m, 5H), 2.35 (s, 3H).

### Beispiel 23A

### 1-(3-Chlorphenyl)-5-(3-cyanophenyl)-1H-pyrazol-3-carbonsäure

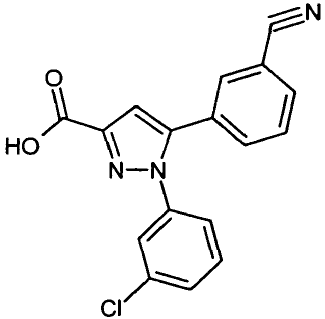

Ausgehend von 2.75 g (7.82 mmol) 1-(3-Chlorphenyl)-5-(3-cyanophenyl)-1H-pyrazol-3-carbonsäureethylester aus Beispiel 16A und 4.39 g (78.2 mmol) Kaliumhydroxid werden nach dem in Beispiel 7A beschriebenen Verfahren 2.37 mg (7.3 mmol, 94% Ausbeute d. Th.) als Kristalle erhalten.
HPLC (Methode 1): Rₜ = 4.20 min
MS (ESIpos): m/z = 324 (M+H)⁺
¹H-NMR (400 MHz, DMSO-d₆): δ = 13.15 (s, 1H), 7.91-7.84 (m, 2H), 7.62-7.43 (m, 5H), 7.27-7.22 (m, 2H).

### Beispiel 24A

### 1-(3-Chlorphenyl)-5-phenyl-1H-pyrazol-3-carbonsäure

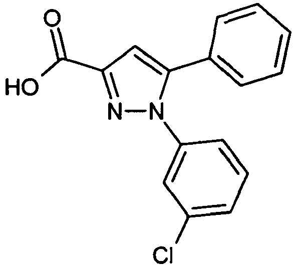

Ausgehend von 400 mg (1.22 mmol) 1-(3-Chlorphenyl)-5-phenyl-1H-pyrazol-3-carbonsäureethylester aus Beispiel 19A und 686.8 mg (12.24 mmol) Kaliumhydroxid werden nach dem in Beispiel 7A beschriebenen Verfahren 335.8 mg (1.1 mmol, 92% Ausbeute d. Th.) als Kristalle erhalten.
Schmelzpunkt: 187°C
HPLC (Methode 2): Rₜ = 4.40 min
MS (ESIpos): m/z = 299 (M+H)⁺
¹H-NMR (400 MHz, DMSO-d₆): δ = 13.06 (s, 1H), 7.55-7.35 (m, 6H), 7.33-7.2 (m, 3H), 7.08 (s, 1H).

### Beispiel 25A

### 1-(3-Chlorphenyl)-5-(3,4-dimethoxyphenyl)-1H-pyrazol-3-carbonsäure

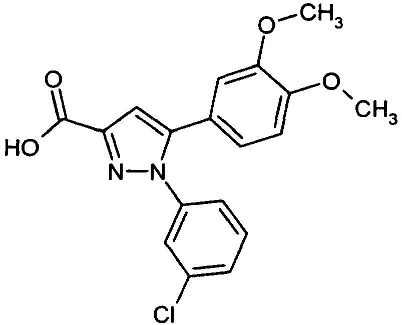

Ausgehend von 500 mg (1.29 mmol) 1-(3-Chlorphenyl)-5-(3,4-dimethoxyphenyl)-1H-pyrazol-3-carbonsäureethylester aus Beispiel 17A und 725.2 mg (12.9 mmol) Kaliumhydroxid werden nach dem in Beispiel 7A beschriebenen Verfahren 434.1 mg (1.2 mmol, 94% Ausbeute d. Th.) als Kristalle erhalten.
Schmelzpunkt: 161°C
HPLC (Methode 2): Rₜ = 4.15 min
MS (ESIpos): m/z = 359 (M+H)⁺
¹H-NMR (400 MHz, DMSO-d₆): δ = 13.1 (s, 1H), 7.57-7.42 (m, 3H), 7.26 (d, 1H), 7.06 (s, 1H), 6.95 (d, 1H), 6.86 (s, 1H), 6.78 (d, 1H), 5.57 (s, 3H), 3.61 (s, 3H).

### Beispiel 26A

### 1-(3-Chlorphenyl)-5-(2,4-dichlorphenyl)-1 H-pyrazol-3-carbonsäure

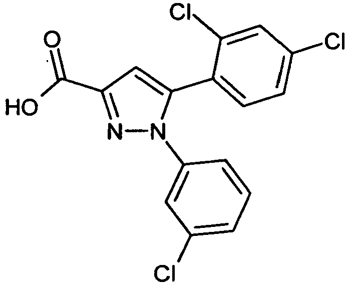

Ausgehend von 240 mg (0.61 mmol) 1-(3-Chlorphenyl)-5-(2,4-dichlorphenyl)-1H-pyrazol-3-carbonsäureethylester aus Beispiel 18A und 340.3 mg (6.1 mmol) Kaliumhydroxid werden nach dem in Beispiel 7A beschriebenen Verfahren 204 mg (0.55 mmol, 92% Ausbeute d. Th.) als Kristalle erhalten.
Schmelzpunkt: 191°C
HPLC (Methode 2): Rₜ = 4.87 min
¹H-NMR (400 MHz, DMSO-d₆): δ = 13.17 (s, 1H), 7.75 (s, 1H), 7.63-7.52 (m, 2H), 7.52-7.36 (m, 3H), 7.16 (d, 1H), 7.07 (s, 1H).

### Beispiel 27A

### 1-(3-Chlorphenyl)-5-(3-nitrophenyl)-1H-pyrazol-3-carbonsäure

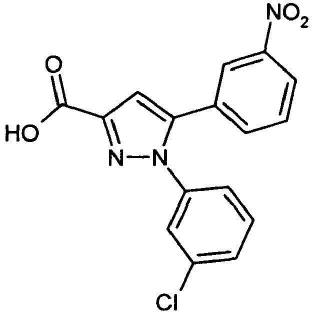

Ausgehend von 400 mg (1.1 mmol) 1-(3-Chlorphenyl)-5-(3-nitrophenyl)-1H-pyrazol-3-carbonsäureethylester aus Beispiel 20A und 603 mg (10.7 mmol) Kaliumhydroxid werden nach dem in Beispiel 7A beschriebenen Verfahren 340.5 mg (0.99 mmol, 92% Ausbeute d. Th.) als Kristalle erhalten.
Schmelzpunkt: 187°C
HPLC (Methode 2): Rₜ = 4.37 min
MS (ESIpos): m/z = 344 (M+H)⁺
¹H-NMR (400 MHz, DMSO-d₆): δ = 13.16 (s, 1H), 8.24 (d, 1H), 8.14 (s, 1H), 7.77-7.41 (m, 5H), 7.36-7.21 (m, 2H).

### Beispiel 28A

### 1-(3-Methoxyphenyl)-5-[3-(trifluormethyl)phenyl]-1H-pyrazol-3-carbonsäure

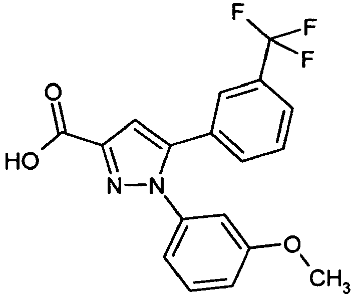

Ausgehend von 1.6 g (4.1 mmol) 1-(3-Methoxyphenyl)-5-[3-(trifluormethyl)phenyl]-1H-pyrazol-3-carbonsäureethylester aus Beispiel 21A und 2.3 g (41 mmol) Kaliumhydroxid werden nach dem in Beispiel 7A beschriebenen Verfahren 1.07 g (2.9 mmol, 72% Ausbeute d. Th.) als Kristalle erhalten.
Schmelzpunkt: 154°C
LC-MS (Methode 3): Rₜ = 2.37 min
MS (ESIpos): m/z = 363 (M+H)⁺
¹H-NMR (400 MHz, DMSO-d₆): δ = 7.77-7.71 (m, 1H), 7.65-7.57 (m, 3H), 7.36 (t, 1H), 7.25 (s, 1H), 7.07-7.02 (m, 1H), 6.96 (s, 1H), 6.86 (d, 1H), 3.71 (s, 3H).

### Ausführungsbeispiele

### Beispiel 1

### 4-({1-(3-Chlorphenyl)-5-[3-(trifluormethyl)phenyl]-1H-pyrazol-3-yl}carbonyl)-morpholin

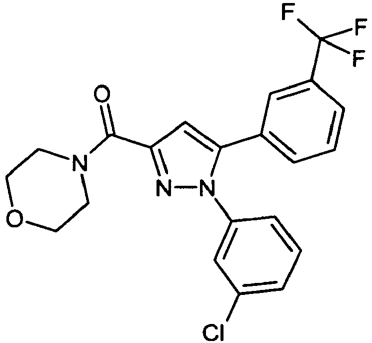

Eine Lösung aus 100 mg (0.27 mmol) 1-(3-Chlorphenyl)-5-[3-(trifluormethyl)phenyl]-1H-pyrazol-3-carbonsäure aus Beispiel 9A in 3 ml Toluol wird unter Sauerstoffausschluss mit 0.06 ml (0.82 mmol) Thionylchlorid versetzt und 3 Stunden bei Rückfluss gerührt. Nach dem Abkühlen wird das Reaktionsgemisch einrotiert. Das erhaltene Zwischenprodukt wird in 2 ml Dichlormethan aufgenommen und auf ca. 0°C abgekühlt. Man gibt 0.06 ml (0.44 mmol) Triethylamin und 0.04 ml (0.44 mmol) Morpholin zu und rührt 12 Stunden bei Raumtemperatur nach. Das Reaktionsgemisch wird auf Essigsäureethylester gegeben und zweimal mit Natriumchlorid-Lösung gewaschen. Die organische Phase wird über Natriumsulfat getrocknet und einrotiert. Der erhaltene Rückstand wird über eine Kieselgelflash (Laufmittel: Cyclo-hexan/ Essigsäureethylester 1:1) getrennt. Das erhaltene Öl wird aus Diethylether/Pentan kristallisiert. Die Kristalle werden abgesaugt und getrocknet. Es werden 92.8 mg (0.21mmol, 78% Ausbeute d. Th.) Produkt erhalten.
HPLC (Methode1) Rₜ = 4.87 min
MS (ESIpos): m/z = 436 (M+H)⁺
¹H-NMR (300 MHz, DMSO-d₆): δ = 7.77 (d, 1H), 7.68-7.42 (m, 6H), 7.28 (d, 1H), 7.15 (s, 1H), 4.0 (s, 2H), 3.75-3.57 (m, 6H).

Analog zu Beispiel 1 werden die folgenden Verbindungen hergestellt:

| **Bsp.-Nr.** | **Struktur** | **hergestellt aus Ausbeute** | **Analytische Daten HPLC/LC-MS (Methode) MS, ¹H-NMR** |
|---|---|---|---|
| **2** | 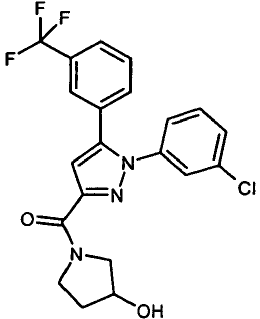 | Beispiel 7A 43 mg, 72% d. Th. | HPLC (1): Rₜ = 2.22 min |
| | | | MS (ESIpos): m/z = 436 (M+H)⁺ |
| | | | ¹H-NMR (300 MHz, CDCl₃): δ = 7.62 (d, 1H), 7.55-7.28 (m, 6H), 7.16-7.01 (m, 2H), 4.66-4.55 (m, 1H), 4.21-4.08 (m, 2H), 3.95-3.75 (m, 2H), 2.17-1.95 (m, 2H). |
| **3** | 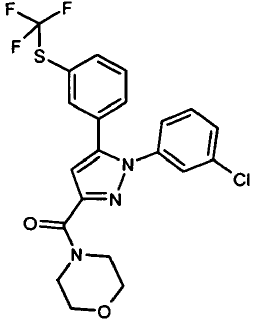 | Beispiel 8A 153.1 mg, 87% d. Th. | HPLC (1): Rₜ = 5.03 min |
| | | | MS (ESIpos): m/z = 468 (M+H)⁺ |
| | | | ¹H-NMR (300 MHz, DMSO-d₆): δ = 7.74 (d, 1H), 7.68-7.41 (m, 6H), 7.28 (d, 1H), 7.08 (s, 1H), 4.02-3.92 (m, 2H), 3.73-3.57 (m, 6H). |
| **4** | 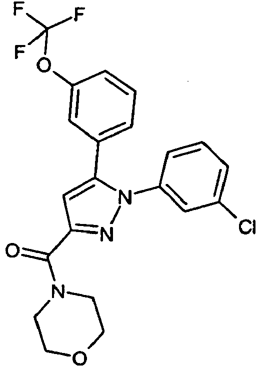 | Beispiel 9A 77.6 mg, 66% d. Th. | LC-MS (3): Rt = 2.47 min |
| | | | MS (ESIpos): m/z = 452 (M+H)+ |
| | | | 1H-NMR (400 MHz, DMSO-d₆): δ = 7.59-7.44 (m, 4H), 7.44-7.38 (m, 2H), 7.28 (d, 1H), 7.18 (s, 1H), 7.08 (s, 1H), 3.99-3.94 (m, 2H), 3.7-3.6 (m, 6H). |
| **5** | 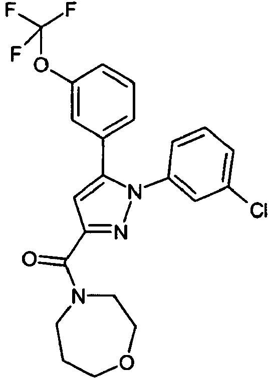 | Beispiel 9A 52.8 mg, 62% d. Th. | LC-MS (2): Rₜ = 2.77 min |
| | | | MS (ESIpos): m/z = 466 (M+H)⁺ |
| | | | ¹H-NMR (400 MHz, CDCl₃): δ = 7.38-7.12 (m, 6H), 7.05-6.96 (m, 2H), 6.95-6.91 (m, 1H), 4.11-4.01 (m, 2H), 3.87-3.71 (m, 6H), 2.05-1.97 (m, 2H). |
| **6** | 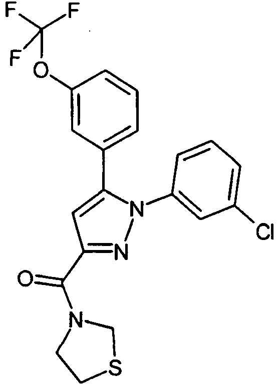 | Beispiel 9A 45.3 mg, 55% d. Th. | LC-MS (3): Rt = 2.98 min |
| | | | MS (ESIpos): m/z = 454 (M+H)⁺ |
| | | | 1H-NMR (400 MHz, CDCl3): δ = 7.46-7.17 (m, 6H), 7.14-6.98 (m, 3H), 5.14 (s, 1H), 4.83 (s, 1H), 4.45-4.35 (m, 1H), 4.13 (m, 1H), 3.18-3.03 (m, 2H). |
| **7** | 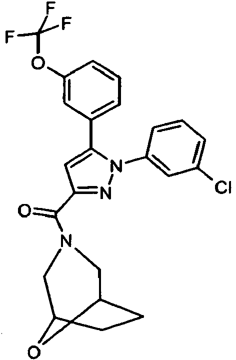 | Beispiel 9A 51 mg, 58% d. Th. | LC-MS (3): Rₜ = 2.84 min |
| | | | MS (ESIpos): m/z = 478 (M+H)⁺ |
| | | | ¹H-NMR (400 MHz, CDCl₃): δ = 7.45-7.16 (m, 6H), 7.08 (s, 2H), 7.04 (s, 1H), 5.4-5.32 (m, 1H), 4.86 (m, 1H), 3.93-3.82 (m, 2H), 3.77-3.66 (m, 2H), 2.18-1.94 (m, 4H). |
| **8** | 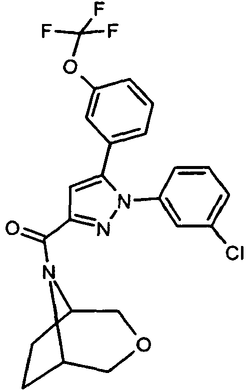 | Beispiel 9A 53.4 mg, 61% d. Th. | LC-MS (2): Rₜ = 2.87 min |
| | | | MS (ESIpos): m/z = 478 (M+H)⁺ |
| | | | ¹H-NMR (400 MHz, CDCl₃): δ = 7.46-7.14 (m, 6H), 7.12-6.9 (m, 3H), 4.61 (d, 1H), 4.53-4.31 (m, 3H), 3.57 (d, 1H), 3.17 (d, 1H), 1.95 (s, 4H). |
| **9** | 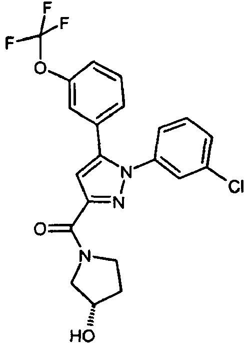 | Beispiel 9A 50.5 mg, 61% d. Th. | LC-MS (3): Rₜ = 2.44 min |
| | | | MS (ESIpos): m/z = 452 (M+H)⁺ |
| | | | ¹H-NMR (400 MHz, CDCl₃): δ = 7.45-77.3 (m, 3H), 7.28-7.18 (m, 3H), 7.12-7.0 (m, 3H), 4.6 (d, 1H), 4.2-4.1 (m, 2H), 3.92-3.85 (m, 1H), 3.8 (s, 1H), 2.17-1.98 (m, 2H). |
| **10** | 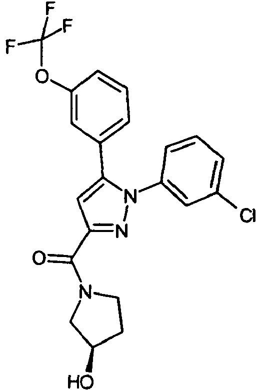 | Beispiel 9A 42 mg, 51% d. Th. | LC-MS (2): Rₜ = 2.48 min |
| | | | MS (ESIpos): m/z = 452 (M+H)⁺ |
| | | | Drehwert (Methanol): α= -24.3° |
| | | | ¹H-NMR (400 MHz, CDCl₃): δ = 7.43-7.3 (m, 3H), 7.3-7.2 (m, 3H), 7.15-7.0 (m, 3H), 4.62-4.55 (m, 1H), 4.32-4.1 (m, 2H), 3.92-3.78 (m, 2H), 2.18-1.98 (m, 2H). |
| **11** | 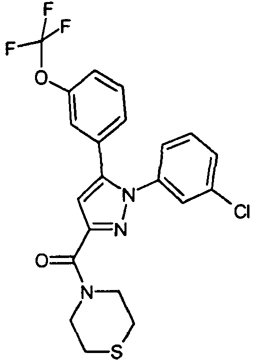 | Beispiel 9A 97.1 mg, 57% d. Th. | LC-MS (1): Rₜ = 2.77 min |
| | | | MS (ESIpos): m/z = 468 (M+H)⁺ |
| | | | ¹H-NMR (400 MHz, DMSO-d₆): δ = 7.59-7.38 (m, 6H), 7.28 (d, 1H), 7.18 (s, 1H), 7.07 (s, 1H), 4.12 (s, 1H, breit), 3.92 (s, 1H, breit), 2.72-2.66 (m, 4H). |
| **12** | 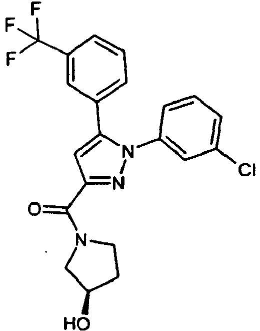 | Beispiel 7A 39 mg, 47% d. Th | LC-MS (2): Rₜ = 2.42 min |
| | | | MS (ESIpos): m/z = 436 (M+H)⁺ |
| | | | ¹H-NMR (400 MHz, CDCl₃): δ = 7.65-7.0 (m, 9H), 4.65-4.55 (m, 1H), 4.32-4.1 (m, 2H), 3.9-3.75 (m, 2H), 2.15-1.95 (m, 2H). |
| **13** | 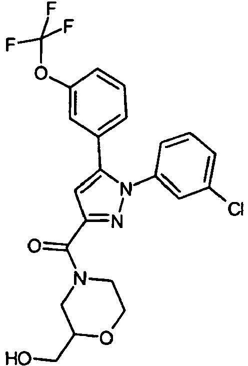 | Beispiel 9A, Amin eingesetzt als 2-({[tert-Butyl(dimethyl)silyl]oxy}-methyl)morpholin 38.1 mg, 20% d Th. | LC-MS (1): Rₜ = 2.25 min |
| | | | MS (ESIpos): m/z = 482 (M-C₆H₁₄Si)⁻ |
| | | | ¹H-NMR (400 MHz, DMSO-d₆): δ = 7.45-7.2 (m, 7H), 7.12-6.99 (m, 2H), 4.9 (d, 0.5H), 4.75 (d, 0.5H), 4.6 (d, 0.5 H), 4.44 (d, 0.5H), 4.01 (t, 1H), 3.83-3.6 (m, 4H) 3.53-3.4 (m, 1H), 3.25 (t, 0.5H), 2.92 (t, 0.5H), 2.19 (t, 0.5H), 1.93 (t, 0.5H). |

### Beispiel 14

### 1-{[1-(3-Chlorphenyl)-5-(3-trifluormethoxyphenyl)-1H-pyrazol-3-yl]carbonyl}piperazin

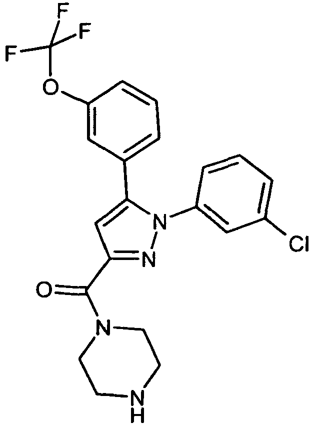

140 mg (0.25 mmol) 4-{[1-(3-Chlorphenyl)-5-(3-trifluormethoxyphenyl)-1H-pyrazol-3-yl]-carbonyl}piperazin-1-carbonsäure-tert.-butylester, hergestellt aus der Verbindung des Beispiels 9A und Piperazin-1-carbonsäure-tert.-butylester in Analogie zu Beispiel 1, wurden unter Sauerstoffausschluss mit 5 ml Dichlormethan und 1 ml Trifluoressigsäure versetzt und 12 Stunden bei Raumtemperatur gerührt. Das Reaktionsgemisch wird mit Dichlormethan verdünnt und mit NatriumhydrogencarbonatLösung und Natriumchlorid-Lösung gewaschen. Die organische Phase wird über Natriumsulfat getrocknet und einrotiert. Es werden 101 mg (88% Ausbeute d. Th.) Produkt erhalten. Eine analytische Probe wird durch scharfes Trocknen im Hochvakuum bei 60°C von Lösemittelresten befreit.
LC-MS (Methode 2): Rₜ = 1.92min
MS (ESIpos): m/z = 451 (M+H)⁺
¹H-NMR (400 MHz, DMSO-d₆): δ = 7.59-7.38 (m, 6H), 7.27 (d, 1H), 7.17 (s, 1H), 7.04 (s, 1H), 3.83 (s, 2H), 3.59 (s, 2H), 279-2.69 (m, 4H).

### Beispiel 15

### 4-{[1-(3-Chlorphenyl)-5-(3-trifluormethylthiophenyl)-1H-pyrazol-3-yl]carbonyl}-piperazin-1-carbaldehyd

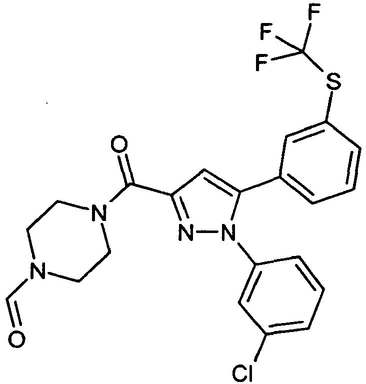

Eine Lösung aus 35.0 mg (0.07 mmol) 1-{[1-(3-Chlorphenyl)-5-(3-trifluormethythio-phenyl)-1H-pyrazol-3-yl]carbonyl}piperazin, hergestellt aus der Verbindung des Beispiels 8A und Piperazin in Analogie zu Beispiel 1, wird in 1 ml DMF unter Sauerstoffausschluss mit 17.2 mg (0.09 mmol) EDC und 11.7 mg (0.09 mmol) HOBt und 8.3 mg (0.18 mmol) Ameisensäure versetzt und 72 Stunden bei Raumtemperatur gerührt. Das Reaktionsgemisch wird über eine präparative HPLC getrennt. Es werden 15.7 mg (42% Ausbeute d. Th.) Produkt erhalten.
LC-MS (Methode 2): Rₜ = 2.6 min
MS (ESIpos): m/z = 495 (M+H)⁺
¹H-NMR (400 MHz, CDCl₃): δ = 8.14 (s, 1H), 7.66 (d, 1H), 7.51-7.23 (m, 6H), 7.11-6.98 (m, 2H), 4.19 (d, 2H), 3.85 (d, 2H), 3.69 (s, 2H), 3.51 (s, 2H)

### Beispiel 16 und Beispiel 17

Ausgehend von 100 µmol der entsprechenden Pyrazolcarbonsäure werden in Analogie zu Beispiel 1 die in der Tabelle aufgeführten Verbindungen hergestellt (Amidkupplung mittels TBTU, Lösemittel DMF, Aufreinigung des Rohprodukts über präparative HPLC, Nachweis der jeweiligen Molmasse als [M+H]⁺):

| **Beispiel-Nr.** | **Struktur** | **Molmasse** | **Rₜ [min]** | **LC/MS-Methode** |
|---|---|---|---|---|
| **16** | 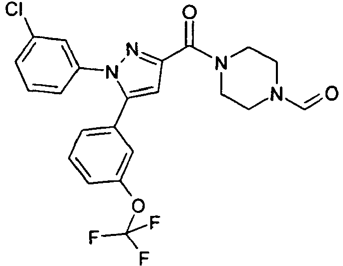 | 478.1 | 2.17 | **4** |
| **17** | 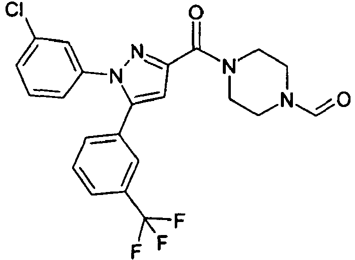 | 462.9 | 2.15 | 4 |

Analog zu Beispiel 1 werden die folgenden Verbindungen hergestellt:

| **Bsp.-Nr.** | **Struktur** | **hergestellt aus Ausbeute** | **Analytische Daten HPLC/LC-MS (Methode) MS, ¹H-NMR** |
|---|---|---|---|
| **18** | 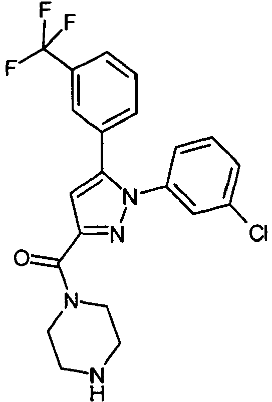 | Beispiel 7A 51.7 mg, 85% d. Th. | LC-MS (1): Rₜ = 1.62 min |
| | | | MS (ESIpos): m/z = 434 (M+H)⁺ |
| | | | ¹H-NMR (400 MHz, CDCl₃): δ = 7.57 (d, 1H), 7.45 (s, 1H), 7.41 (t, 1H), 7.36-7.25 (m, 3H), 7.23-7.15 (m, 1H), 6.99 (d, 1H), 6.92 (s, 1H), 4.03 (s, 2H), 3.78 (s, 2H), 3.0-2.85 (m, 4H). |
| **19** | 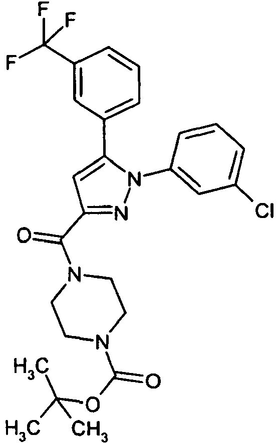 | Beispiel 7A 85 mg, 73% d. Th. | LC-MS (2): Rₜ = 3.09 min |
| | | | MS (ESIpos): m/z = 535 (M+H)⁺ |
| | | | ¹H-NMR (300 MHz, CDCl₃): δ = 7.63 (d, 1H), 7.56-7.44 (m, 2H), 7.42-7.33 (m, 3H), 7.31-7.27 (m, 1H), 7.06 (d, 1H), 7.0 (s, 1H), 4.13-4.05 (m, 2H), 3.85-3.75 (m, 2H), 3.6-3.48 (m, 4H), 1.48 (s, 9H). |
| **20** | 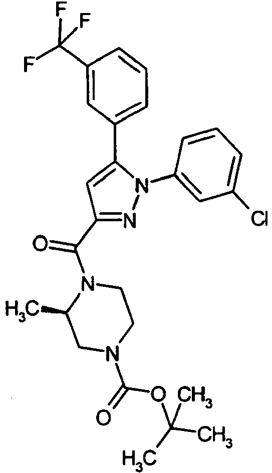 | Beispiel 7A 165.3 mg, 74% d. Th. | HPLC (1): Rₜ = 5.64 min |
| | | | MS (ESIpos): m/z = 549 (M+H)⁺ |
| | | | ¹H-NMR (400 MHz, DMSO-d₆): δ = 7.76 (d, 1H), 7.67-7.56 (m, 3H), 7.52 (d, 2H), 7.46 (t, 1H), 7.27 (d, 1H), 7.11 (s, 1H), 4.88-4.68 (m, 1H), 4.51-4.27 (m, 1H), 4.07-3.74 (m, 2H), 3.17-2.76 (m, 3H), 1.42 (s, 9H), 1.3-1.12 (m, 3H). |
| **21** | 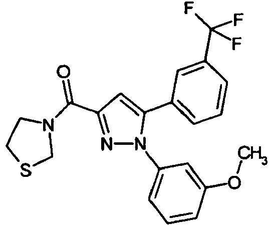 | Beispiel 28A 57.6 mg, 69% d. Th. | LC-MS (2): Rₜ = 2.91 min |
| | | | MS (ESIpos): m/z = 434 (M+H)⁺ |
| | | | ¹H-NMR (400 MHz, DMSO-d₆): δ = 7.74 (d, 1H), 7.65-7.57 (m, 3H), 7.36 (t, 1H), 7.21 (s, 1H), 7.06-6.95 (m, 2H), 6.89 (d, 1H), 5.05 (s, 1H), 4.7 (s, 1H), 4.27-4.21 (m, 1H), 3.91-3.84 (m, 1H), 3.7 (s, 3H), 3.15-3.03 (m, 2H). |
| **22** | 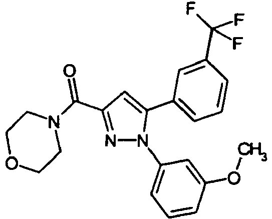 | Beispiel 28A 63.8 mg, 77% d Th. | LC-MS (1): Rₜ = 2.3 min |
| | | | MS (ESIpos): m/z = 432 (M+H)⁺ |
| | | | ¹H-NMR (400 MHz, DMSO-d₆): δ = 7.74 (d, 1H), 7.65-7.56 (m, 3H), 7.35 (t, 1H), 7.11 (s, 1H), 7.02 (d, 1H), 6.94 (s, 1H), 6.86 (d, 1H), 3.99 (s, 2H), 3.73-3.58 (m, 9H). |
| **23** | 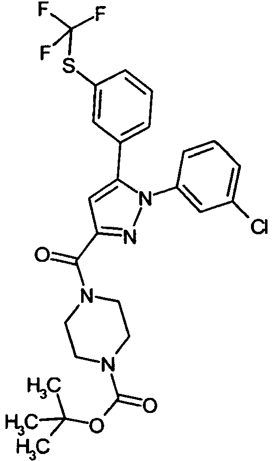 | Beispiel 8A 154.5 mg, 68% d. Th. | LC-MS (2): Rₜ = 3.21 min |
| | | | MS (ESIpos): m/z = 567 (M+H)⁺ |
| | | | ¹H-NMR (300 MHz, CDCl₃): δ = 7.65 (d, 1H), 7.5-7.38 (m, 3H), 7.38-7.3 (m, 3H), 7.06 (d, 1H), 6.98 (s, 1H), 4.15-4.05 (m, 2H), 3.85-3.74 (m, 2H), 3.6-3.47 (m, 4H), 1.48 (s, 9H). |
| **24** | 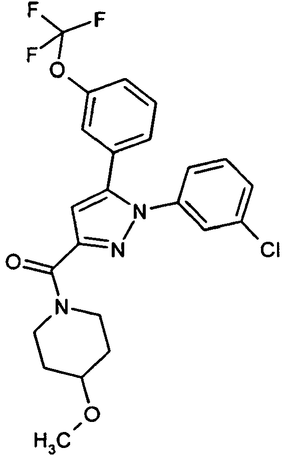 | Beispiel 9A 46.7 mg, 53% d. Th. | LC-MS (1): Rₜ = 2.65 min |
| | | | MS (ESIpos): m/z = 480 (M+H)⁺ |
| | | | ¹H-NMR (400 MHz, DMSO-d₆): δ = 7.44-7.16 (m, 6H), 7.12-7.0 (m, 2H), 6.93 (s, 1H), 4.37-4.22 (m, 1H), 4.18-4.02 (m, 1H), 3.84-3.68 (m, 1H), 3.63-3.46 (m, 2H), 3.39 (s, 3H), 2.04-1.87 (m, 2H), 1.78-1.56 (m, 2H). |
| **25** | 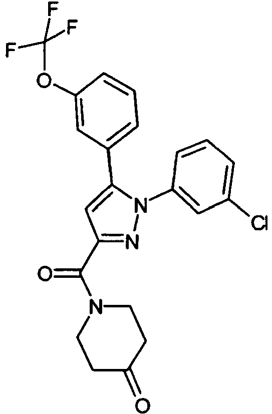 | Beispiel 9A 69.9 mg, 38% d. Th. | LC-MS (3): Rₜ = 2.65 min |
| | | | MS (ESIpos): m/z = 464 (M+H)⁺ |
| | | | ¹H-NMR (400 MHz, CDCl₃): δ = 7.45-7.34 (m, 3H), 7.32-7.2 (m, 3H), 7.09 (d, 1H), 7.04 (s, 2H), 4.45-4.35 (m, 2H), 4.12-4.03 (m, 2H), 2.66-2.56 (m, 4H). |
| **26** | 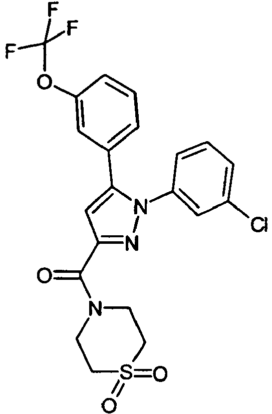 | Beispiel 9A 50.9 mg, 56% d. Th. | LC-MS (2): Rₜ = 2.69 min |
| | | | MS (ESIpos): m/z = 500 (M+H)⁺ |
| | | | ¹H-NMR (400 MHz, EDCl₃): δ = 7.45-7.36 (m, 2H), 7.36-7.17 (m, 4H), 7.12-7.0 (m, 3H), 4.65 (s, 2H), 4.29 (s, 2H), 3.2 (d, 4H). |
| **27** | 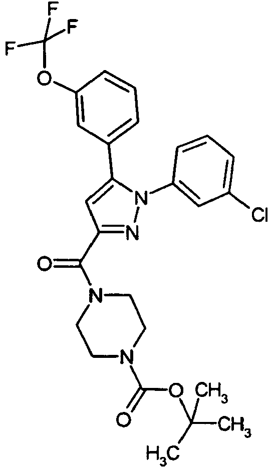 | Beispiel 9A 150 mg, 50% d. Th. | LC-MS (2): Rₜ = 3.15 min |
| | | | MS (ESIpos): m/z = 551 (M+H)⁺ |
| | | | ¹H-NMR (400 MHz, DMSO-d₆): δ = 7.59-7.44 (m, 4H), 7.41 (d, 2H), 7.29 (d, 1H), 7.18 (s, 1H), 7.09 (s, 1H), 3.93 (s, 2H, breit), 3.64 (s, 2H, breit), 3.41 (s, 4H, breit), 1.42 (s, 9H). |
| **28** | 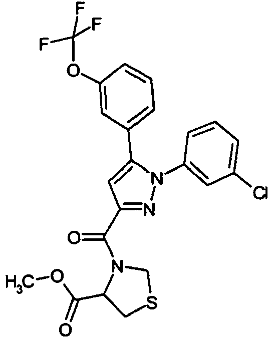 | Beispiel 9A 32 mg, 14% d. Th. | LC-MS (2): Rₜ = 3.03 min |
| | | | MS (ESIpos): m/z = 512 (M+H)⁺ |
| | | | ¹H-NMR (400 MHz, DMSO-d₆): δ = 7.6-7.4 (m, 6H), 7.22-7.12 (m, 3H), 5.8, 5.4, 5.1 (d,d,t,2H, verschiedene Signale durch E/Z-Isomere), 5.0, 4.9, 4.67 (3d, 2H, verschiedene Signale durch E/Z-Isomere), 3.7, 3.6 (2s, 3H, verschiedene Signale durch E/Z-Isomere), 3.58-3.4 (m, 1H). |
| **29** | 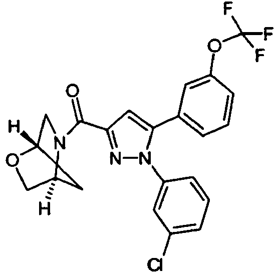 | Beispiel 9A 41 mg, 48% d. Th. | LC-MS (2): Rₜ = 2.71 min |
| | | | MS (ESIpos): m/z = 464 (M+H)⁺ |
| | | | ¹H-NMR (400 MHz, DMSO-d₆): δ = 7.6-7.4 (m, 6H), 7.35-7.25 (m, 1H), 7.2-7.11 (m, 2H), 5.6, 4.95 (2s, 1H, verschiedene Signale durch E/Z-Isomere), 4.66 (s, 1H), 3.9-3.7 (m, 3H), 3.5, 3.4 (2d, 1H, verschiedene Signale durch E/Z-Isomere), 1.95-1.8 (m, 2H). |
| **30** | 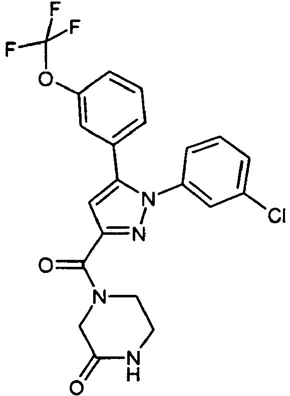 | Beispiel 9A 43.4 mg, 51% d. Th. | LC-MS (2): Rₜ = 2.40 min |
| | | | MS (ESIpos): m/z = 465 (M+H)⁺ |
| **31** | 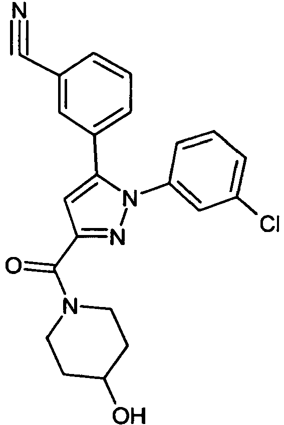 | Beispiel 23A 24.7 mg, 30% d. Th. | LC-MS (3): Rₜ = 2.06 min |
| | | | MS (ESIpos): m/z = 407 (M+H)⁺ |
| | | | ¹H-NMR (400 MHz, CDCl₃): δ = 7.66 (s, 1H), 7.59-7.43 (m, 3H), 7.36 (s, 2H), 7.32-7.23 (m, 1H), 7.05 (d, 1H), 6.95 (s, 1H), 4.5-4.37 (m, 1H), 4.32-4.21 (m, 1H), 4.08-3.97 (m, 1H), 3.73-3.61 (m, 1H), 3.48-3.36 (m, 1H), 2.08-1.94 (m, 2H), 1.71-1.58 (m, 2H). |
| **32** | 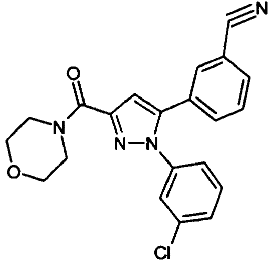 | Beispiel 23A 64.1 mg, 53% d. Th. | HPLC (2): Rₜ = 4.39 min |
| | | | MS (ESIpos): m/z = 393 (M+H)⁺ |
| | | | ¹H-NMR (300 MHz, DMSO-d₆): δ = 7.92-7.84 (m, 2H), 7.63-7.42 (m, 5H), 7.24 (d, 1H), 7.12 (s, 1H), 3.97 (s, 2H), 3.75-3.57 (m, 6H). |
| **33** | 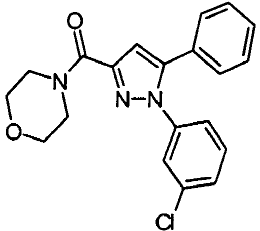 | Beispiel 24A 43.2 mg, 70% d. Th. | HPLC (2): Rₜ = 5.05 min |
| | | | MS (ESIpos): m/z = 368 (M+H)⁺ |
| | | | ¹H-NMR (400 MHz, DMSO-d₆): δ = 7.53-7.35 (m, 6H), 7.33-7.16 (m, 3H), 6.95 (s, 1H), 3.98 (s, 2H), 3.72-3.57 (m, 6H). |
| **34** | 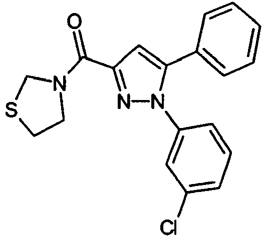 | Beispiel 24A 49.1 mg, 79% d. Th. | HPLC (2): Rₜ = 5.11 min |
| | | | MS (ESIpos): m/z = 370 (M+H)⁺ |
| | | | ¹H-NMR (400 MHz, DMSO-d₆): δ = 7.55-7.37 (m, 6H), 7.32-7.23 (m, 3H), 7.05 (s, 1H), 5.06 (s, 1H), 4.69 (s, 1H), 4.27-4.2 (m, 1H), 3.9-3.83 (m, 1H), 3.16-3.03 (m, 2H). |
| **35** | 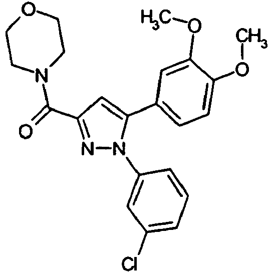 | Beispiel 25A 52.9 mg, 89% d. Th. | HPLC (2): Rₜ = 4.32 min |
| | | | MS (ESIpos): m/z = 428 (M+H)⁺ |
| | | | ¹H-NMR (400 MHz, DMSO-d₆): δ = 7.52-7.43 (m, 3H), 7.26 (d, 1H), 6.98-6.91 (m, 2H), 6.86-6.83 (m, 1H), 6.78 (dd, 1H), 4.01-3.95 (m, 2H), 3.75 (s, 3H), 3.69-3.58 (m, 9H). |
| **36** | 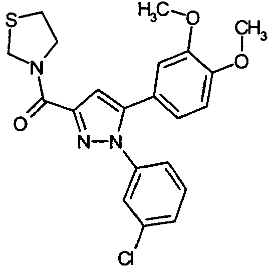 | Beispiel 25A 47.6 mg, 79% d. Th. | HPLC (2): Rₜ = 4.68 min |
| | | | MS (ESIpos): m/z = 430 (M+H)⁺ |
| | | | ¹H-NMR (400 MHz, DMSO-d₆): δ = 7.59-7.44 (m, 3H), 7.28 (d, 1H), 7.03 (s, 1H), 6.96 (d, 1H), 6.87-6.84 (m, 1H), 6.81-6.75 (m, 1H), 5.05 (s, 1H), 4.69 (s, 1H), 4.23 (t, 1H), 3.86 (t, 1H), 3.75 (s, 3H), 3.62 (s, 3H). |
| **37** | 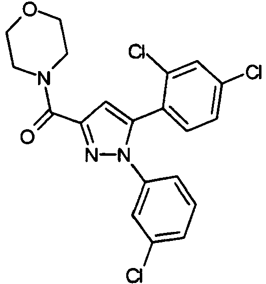 | Beispiel 26A 38.3 mg, 69% d. Th. | HPLC (2): Rₜ = 4.98 min |
| | | | MS (ESIpos): m/z = 436 (M+H)⁺ |
| | | | ¹H-NMR (400 MHz, DMSO-d₆): δ = 7.75 (s, 1H), 7.62-7.53 (m, 2H), 7.48-7.37 (m, 3H), 7.14 (d, 1H), 6.96 (s, 1H), 4.04-3.93 (m, 2H), 3.66 (s, 6H). |
| **38** | 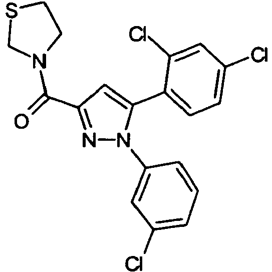 | Beispiel 26A 27.8 mg, 50% d. Th. | HPLC (2): Rₜ = 5.34 min |
| | | | MS (ESIpos): m/z = 438 (M+H)⁺ |
| | | | ¹H-NMR (400 MHz, DMSO-d₆): δ = 7.75 (s, 1H), 7.63-7.53 (m, 2H), 7.51-7.35 (m, 3H), 7.17 (d, 1H), 7.04 (s, 1H), 5.07 (s, 1H), 4.69 (s, 1H), 4.3-4.19 (m, 1H), 3.92-3.83 (m, 1H), 3.19-3.03 (m, 2H). |
| **39** | 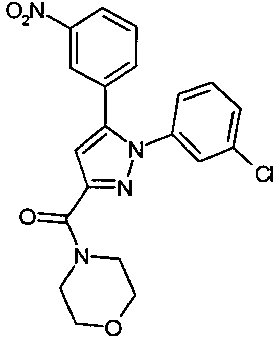 | Beispiel 27A 48.9 mg, 81% d Th. | HPLC (2): Rₜ = 4.45 min |
| | | | MS (ESIpos): m/z = 413 (M+H)⁺ |
| | | | ¹H-NMR (400 MHz, DMSO-d₆): δ = 8.27-8.21 (m, 1H), 8.13 (s, 1H), 7.73-7.65 (m, 2H), 7.59 (s, 1H), 7.54 (d, 1H), 7.4 (t, 1H), 7.29 (t, 1H), 7.18 (s, 1H), 3.97 (s, 2H), 3.72-3.59 (m, 6H). |
| **40** | 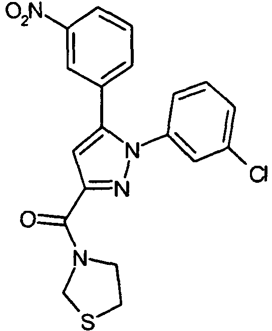 | Beispiel 27A 49.2 mg, 82% d Th. | HPLC (2): Rₜ = 4.78 min |
| | | | MS (ESIpos): m/z = 415 (M+H)⁺ |
| | | | ¹H-NMR (400 MHz, DMSO-d₆): δ = 8.24 (d, 1H), 8.14 (s, 1H), 7.74-7.6 (m, 3H), 7.55 (d, 1H), 7.47 (t, 1H), 7.31 (d, 1H), 7.27 (s, 1H), 5.05 (s, 1H), 4.7 (s, 1H), 4.24 (t, 1H), 3.88 (t, 1H), 3.16-3.04 (m, 2H). |

Analog zu Beispiel 14 werden die folgenden Verbindungen hergestellt:

| **Bsp.-Nr.** | **Struktur** | **hergestellt aus Ausbeute** | **Analytische Daten HPLC/LC-MS (Methode) MS, ¹H-NMR** |
|---|---|---|---|
| **41** | 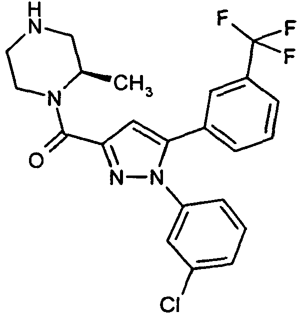 | Beispiel 20 43 mg, 42% d. Th. | HPLC (2): Rₜ = 4.45 min |
| | | | MS (ESIpos): m/z = 449 (M+H)⁺ |
| | | | ¹H-NMR (400 MHz, DMSO-d₆): δ = 7.76 (d, 1H), 7.68-7.41 (m, 6H), 7.26 (d, 1H), 7.07 (s, 1H), 4.68-4.55 (m, 1H), 4.27 (d, 1H), 3.09-2.64 (m, 5H), 1.30 (s, 3H). |

Analog zu Beispiel 15 werden die folgenden Verbindungen hergestellt:

| **Bsp.-Nr.** | **Struktur** | **hergestellt aus Ausbeute** | **Analytische Daten HPLC/LC-MS (Methode) MS** |
|---|---|---|---|
| **42** | 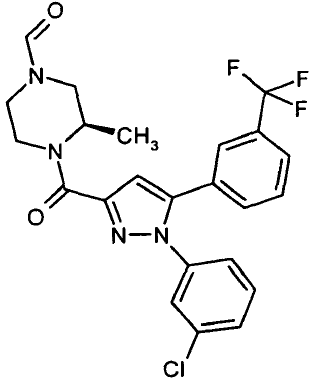 | Beispiel 41 21.4 mg, 71%d.Th. | HPLC (2): Rₜ = 4.69 min |
| | | | MS (ESIpos): m/z = 477 (M+H)⁺ |

Analog zu Beispiel 16 werden die folgenden Verbindungen hergestellt:

| **Beispiel-Nr.** | **Struktur** | **Molmasse** | **Rₜ [min]** | **LC/MS-Methode** |
|---|---|---|---|---|
| **43** | 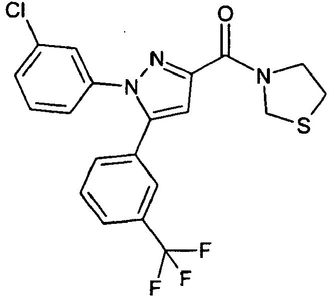 | 437.9 | 2.54 | 4 |
| **44** | 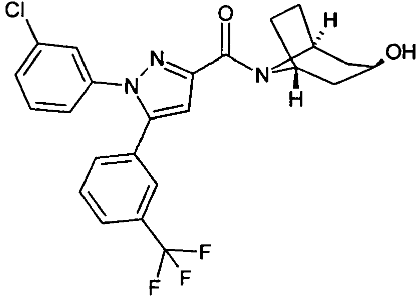 | 475.9 | 2.38 | 4 |
| **45** | 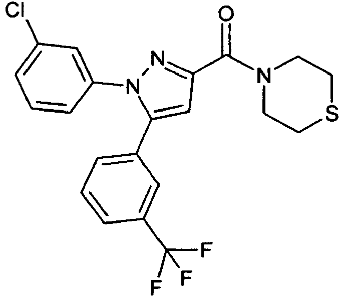 | 451.9 | 2.52 | 4 |
| **46** | 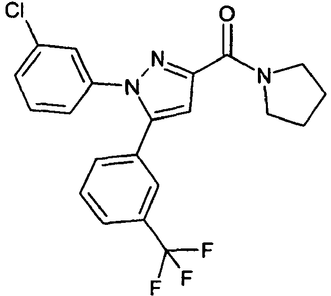 | 419.8 | 2.48 | 4 |
| **47** | 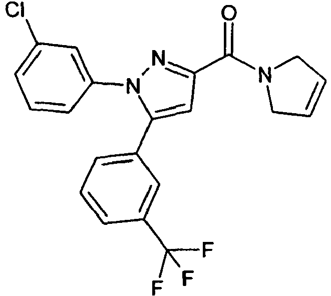 | 417.8 | 2.52 | 4 |

### Beispiel 48

### 4-({1-(3-Chlorphenyl)-5-[3-(trifluormethoxy)phenyl]-1H-pyrazol-3-yl}carbonyl)-thiomorpholin-1-oxid

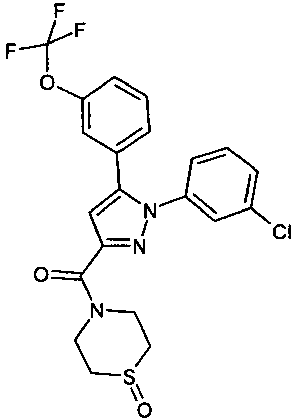

90 mg (0.19 mmol) der Verbindung aus Beispiel 11 in 4 ml Dichlormethan werden bei 0°C mit 57 mg (0.23 mmol) 70%iger 3-Chlorperbenzoesäure versetzt. Nach 2 h Rühren bei dieser Temperatur wird die Mischung nacheinander mit verdünnter Natronlauge und Thiosulfatlösung gewaschen und über Kieselgel (Extrelut) filtriert. Das nach dem Einengen des Eluats verbleibende Öl wird durch präparative HPLC (RP18, Acetonitril/Wasser-Gradient) gereinigt. Man erhält 20 mg, (22% d. Th.) des Produktes.
LC-MS (2): Rₜ = 2.5 min
MS (ESIpos): m/z = 484 (M+H)⁺
¹H-NMR (400 MHz, DMSO-d₆): δ = 7.6-7.45 (m, 4H), 7.42 (d, 2H), 7.29 (d, 1H), 7.18 (s, 1H), 7.1 (s, 1H), 4.6 (d, 1H), 4.39 (d, 1H), 4.07 (t, 1H), 3.73 (t, 1H), 3.06-2.91 (m, 2H), 2.87-2.78 (m, 2H).

Auf gleiche Weise wie Beispiel 48 kann erhalten werden:

| **Bsp.-Nr.** | **Struktur** | **hergestellt aus Ausbeute** | **Analytische Daten HPLC/LC-MS (Methode) MS, ¹H-NMR** |
|---|---|---|---|
| **49** | 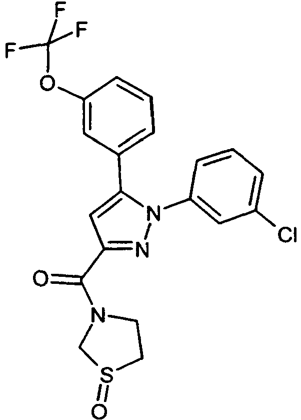 | Beispiel 6 41 mg, 44% d. Th. | LC-MS (2): Rₜ = 2.5 min |
| | | | MS (ESIpos): m/z = 470 (M+H)⁺ |
| | | | ¹H-NMR (400 MHz, DMSO-d₆): δ = 7.63-7.4 (m, 6H), 7.32 (d, 1H), 7.26-7.18 (m, 2H), 5.48, 4.98 (2d, 1H, verschiedene Signale durch E/Z-Isomere), 4.7-4.0 (m, 3H), 3.25-3.0 (m, 2H). |

### B) Bewertung der physiologischen Wirksamkeit

### Abkürzungen:

| | |
|---|---|
| RPMI 1640 | Medium von Gibco, Invitrogen Corporation, Karlsruhe, Deutschland |
| FKS | fötales Kälberserum |

Die Eignung der erfindungsgemäßen Verbindungen zur Behandlung von durch Retroviren hervorgerufenen Erkrankungen kann in folgendem Assay-System gezeigt werden:

### In vitro Assay

### HIV-Infektion in Zellkultur

Der HIV-Test wird mit Modifikationen nach der Methode von Pauswels et al. [vgl. Journal of Virological Methods 1988, 20, 309-321] durchgeführt.

Primäre menschliche Blutlymphozyten (PBL's) werden über Ficoll-Hypaque angereichert und in RPMI 1640 Medium, 20% fötales Kälberserum mit Phythaemagglutinin (90 µg/ml) und Interleukin-2 (40 U/ml) stimuliert. Zur Infektion mit dem infektiösen HIV werden PBL's pelletiert und das Zellpellet anschließend in 1 ml einer geeignet verdünnten HIV-Virusadsorptionslösung suspendiert und 1 Stunde bei 37°C inkubiert (Pelletinfektion). Nicht absorbiertes Virus wird anschließend mittels Zentrifugation entfernt, und die infizierten Zellen werden in Testplatten überführt (z. B. 96-well Mikrotiterplatten), die die Prüfsubstanzen in geeigneter Verdünnung enthalten.

Alternativ werden z.B. HIV-suszeptible, permanente H9-Zellen (ATCC oder NIAID, USA) anstelle von normalen menschlichen Blutlymphozyten zur Testung der antiviralen Effekte der erfindungsgemäßen Verbindungen eingesetzt. Infizierte H9-Zellen werden für Testzwecke in RPMI 1640 Medium, 2% und/oder 20% fötales Kälberserum, gezüchtet.

Die Virusadsorptionslösung wird zentrifugiert und das infizierte Zellpellet in Wachstumsmedium aufgenommen, so dass 1 x 10⁵ Zellen pro ml eingestellt sind. Die derart infizierten Zellen werden mit ca. 1 x 10⁴ Zellen/Napf in die Näpfe von 96er Mikrotiterplatten pipettiert (Pelletinfektion). Alternativ wird das HIV erst nach Zubereitung der Substanzverdünnungen in den Mikrotiterplatten und nach Zugabe der Zellen separat zupipettiert (Überstandsinfektion).

Die erste vertikale Reihe der Mikrotiterplatte enthält nur Wachstumsmedium und Zellen, die nicht infiziert, aber ansonsten genauso wie oben beschrieben, behandelt werden (Zellkontrolle). Die zweite vertikale Reihe der Mikrotiterplatte erhält nur HIVinfizierte Zellen (Viruskontrolle) in Wachstumsmedium. Die übrigen Näpfe enthalten die erfindungsgemäßen Verbindungen in unterschiedlichen Konzentrationen, ausgehend von den Näpfen der 3. vertikalen Reihe der Mikrotiterplatte, von der die Prüfsubstanzen in 2er Schritten 2¹⁰-fach verdünnt werden.

Alternativ werden Überstandsinfektionen durchgeführt (s.o.), bei denen die Zellen in 96-well Platten ausgesät werden. Das HIV-Virus wird dann in einem Volumen von 50 µl zugesetzt.

Die Testansätze werden so lange bei 37°C inkubiert, bis in der unbehandelten Viruskontrolle die für das HIV typische Syncytienbildung auftritt (zwischen Tag 3 und 6 nach Infektion), die dann entweder mikroskopisch oder über p24 ELISA Nachweisverfahren (Vironostika, BioMerieux, Niederlande) oder mittels Alamar Blue Indikator Farbstoff photometrisch oder fluorimetrisch ausgewertet wird. In der unbehandelten Viruskontrolle resultieren unter diesen Testbedingungen etwa 20 - 100 Syncytien, während die unbehandelte Zellkontrolle keine Syncytien aufweist. Entsprechend zeigt der ELISA Test Werte kleiner 0.1 für die Zellkontrollen und Werte zwischen 0.1 und 2.9 für die Viruskontrollen auf. Die photometrische Auswertung der Alamar Blue behandelten Zellen zeigt für die Zellkontrollen Extinktionen kleiner 0.1 auf, während die Viruskontrollen Werte zwischen 0.1 und 3 bei entsprechenden Wellenlängen aufweisen.

Die IC₅₀-Werte werden als die Konzentration der Testsubstanz ermittelt, bei der 50% (ca. 20 - 100 Syncytien) der virusinduzierten Syncytien durch die Behandlung mit der erfindungsgemäßen Verbindung unterdrückt sind. Entsprechend werden die Cut off-Werte beim ELISA Test und bei der photometrischen oder fluorimetrischen Bestimmung mittels Alamar Blue gesetzt. Neben der Bestimmung der antiviralen Effekte werden die behandelten Zellkulturen auch hinsichtlich zytotoxischer, zytostatischer oder zytologischer Veränderungen sowie hinsichtlich Löslichkeit mikroskopisch untersucht. Wirkverbindungen, die im Konzentrationsbereich der Wirkung zellverändernde, zytotoxische Befunde zeigen, werden nicht in ihrer antiviralen Wirksamkeit beurteilt.

Es wird gefunden, dass die erfindungsgemäßen Verbindungen HIV infizierte Zellen vor der virusinduzierten Zellzerstörung schützen. Experimentelle Daten sind in Tabelle A zusammengestellt.

**Tabelle A:**

| **Beispiel-Nr.** | **IC₅₀ (µM), H9 Zellen, 2% FCS** |
|---|---|
| **2** | 0.05 |
| **4** | 0.05 |
| **5** | 0.04 |
| **6** | 0.1 |
| **10** | 0.05 |
| **15** | 0.15 |
| **32** | 0.04 |
| **33** | 0.05 |
| **34** | 0.08 |
| **35** | 0.1 |
| **43** | 0.1 |

### C) Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 1, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Lösung:

### Zusammensetzung

500 mg der Verbindung von Beispiel 1, 2.5 g Polysorbat und 97 g Polyethylenglycol 400. Einer Einzeldosis von 100 mg der erfindungsgemäßen Verbindung entsprechen 20 g orale Lösung.

### Herstellung

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v. Lösung:

Die erfindungsgemäße Verbindung wird in einer Konzentration unterhalb der Sättigungslöslichkeit in einem physiologisch verträglichen Lösungsmittel (z.B. isoton. Kochsalzlösung, Glucoselösung 5%, PEG 400 Lösung 30%) gelöst. Die Lösung wird steril filtriert und in sterile und pyrogenfreie Injektionsbehältnisse abgefüllt.

## Patentansprüche

1. Verwendung einer Verbindung der Formel 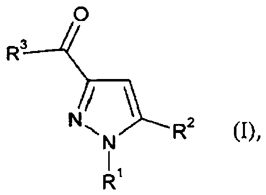 in welcher
R¹ für Phenyl steht,
wobei Phenyl substituiert ist mit 1 bis 3 Substituenten, wobei die Substituenten unabhängig voneinander ausgewählt werden aus der Gruppe bestehend aus Halogen, Cyano, Nitro, Trifluormethyl, Trifluormethoxy, Trifluormethylthio, C₁-C₄-Alkyl und C₁-C₄-Alkoxy,
R² für Phenyl steht,
wobei Phenyl substituiert sein kann mit 1 bis 3 Substituenten, wobei die Substituenten unabhängig voneinander ausgewählt werden aus der Gruppe bestehend aus Halogen, Cyano, Nitro, Trifluormethyl, Trifluormethoxy, Trifluormethylthio, C₁-C₄-Alkyl und C₁-C₄-Alkoxy,
R³ für einen über Stickstoff gebundenen 5- bis 8-gliedrigen Heterocyclus steht,
wobei der Heterocyclus substituiert sein kann mit 1 bis 3 Substituenten, wobei die Substituenten unabhängig voneinander ausgewählt werden aus der Gruppe bestehend aus Halogen, Hydroxy, Hydroxymethyl, Formyl, Amino, Oxo, Trifluormethyl, Trifluormethoxy, C₁-C₄-Alkyl, C₁-C₄-Alkoxy und C₁-C₄-Alkoxycarbonyl,
oder eines ihrer Salze, ihrer Solvate oder der Solvate ihrer Salze,
zur Herstellung eines Arzneimittels zur Behandlung und/oder Prophylaxe von retroviralen Erkrankungen.

2. Verwendung einer Verbindung nach Anspruch 1, **dadurch gekennzeichnet, dass**
R¹ für Phenyl steht,
wobei Phenyl substituiert ist mit 1 bis 3 Substituenten, wobei die Substituenten unabhängig voneinander ausgewählt werden aus der Gruppe bestehend aus Halogen, Cyano, Nitro, Trifluormethyl, Trifluormethoxy, Trifluormethylthio, C₁-C₄-Alkyl und C₁-C₄-Alkoxy,
R² für Phenyl steht,
wobei Phenyl substituiert sein kann mit 1 bis 3 Substituenten, wobei die Substituenten unabhängig voneinander ausgewählt werden aus der Gruppe bestehend aus Halogen, Cyano, Nitro, Trifluormethyl, Trifluormethoxy, Trifluormethylthio, C₁-C₄-Alkyl und C₁-C₄-Alkoxy,
R³ für Pyrrolidin-1-yl, Morpholin-4-yl, Thiomorpholin-4-yl, 1,3-Thiazolidin-3-yl, Piperidin-1-yl, Piperazin-1-yl oder 1,4-Oxazepan-4-yl steht,
wobei Pyrrolidin-1-yl, Morpholin-4-yl, Thiomorpholin-4-yl, 1,3-Thiazolidin-3-yl, Piperidin-1-yl, Piperazin-1-yl oder 1,4-Oxazepan-4-yl substituiert sein können mit 1 bis 3 Substituenten, wobei die Substituenten unabhängig voneinander ausgewählt werden aus der Gruppe bestehend aus Hydroxy, Hydroxymethyl, Formyl, Amino, Oxo, C₁-C₄-Alkyl, C₁-C₄-Alkoxy und C₁-C₄-Alkoxycarbonyl.

3. Verwendung einer Verbindung nach Anspruch 1 oder 2, **dadurch gekennzeichnet, dass**
R¹ für Phenyl steht,
wobei Phenyl substituiert ist mit 1 bis 3 Substituenten, wobei die Substituenten unabhängig voneinander ausgewählt werden aus der Gruppe bestehend aus Halogen, Cyano, Nitro, Trifluormethyl, Trifluormethoxy, Trifluormethylthio, C₁-C₄-Alkyl und C₁-C₄-Alkoxy,
R² für Phenyl steht,
wobei Phenyl substituiert sein kann mit 1 bis 3 Substituenten, wobei die Substituenten unabhängig voneinander ausgewählt werden aus der Gruppe bestehend aus Halogen, Cyano, Nitro, Trifluormethyl, Trifluormethoxy, Trifluormethylthio, C₁-C₄-Alkyl und C₁-C₄-Alkoxy,
und
R³ für einen über Stickstoff gebundenen 5- bis 8-gliedrigen Heterocyclus steht,
wobei der Heterocyclus substituiert ist mit 1 bis 3 Substituenten, wobei die Substituenten unabhängig voneinander ausgewählt werden aus der Gruppe bestehend aus Halogen, Hydroxy, Hydroxymethyl, Formyl, Amino, Oxo, Trifluormethyl, Trifluormethoxy, C₁-C₄-Alkyl, C₁-C₄-Alkoxy und C₁-C₄₋Alkoxycarbonyl,
oder
R¹ für Phenyl steht,
wobei Phenyl substituiert ist mit 1 bis 3 Substituenten, wobei die Substituenten unabhängig voneinander ausgewählt werden aus der Gruppe bestehend aus Halogen, Cyano, Nitro, Trifluormethyl, Trifluormethoxy, Trifluormethylthio, C₁-C₄-Alkyl und C₁-C₄-Alkoxy,
R² für Phenyl steht,
wobei Phenyl substituiert sein kann mit 1 bis 3 Substituenten, wobei die Substituenten unabhängig voneinander ausgewählt werden aus der Gruppe bestehend aus Trifluormethoxy und Trifluormethylthio,
und
R³ für einen über Stickstoff gebundenen unsubstituierten 5- bis 8-gliedrigen Heterocyclus steht.

4. Verbindung der Formel 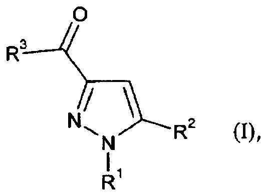 in welcher
R¹ für Phenyl steht,
wobei Phenyl substituiert ist mit 1 bis 3 Substituenten, wobei die Substituenten unabhängig voneinander ausgewählt werden aus der Gruppe bestehend aus Halogen, Cyano, Nitro, Trifluormethyl, Trifluormethoxy, Trifluormethylthio, C₁-C₄-Alkyl und C₁-C₄-Alkoxy,
R² für Phenyl steht,
wobei Phenyl substituiert sein kann mit 1 bis 3 Substituenten, wobei die Substituenten unabhängig voneinander ausgewählt werden aus der Gruppe bestehend aus Halogen, Cyano, Nitro, Trifluormethyl, Trifluormethoxy, Trifluormethylthio, C₁-C₄-Alkyl und C₁-C₄-Alkoxy,
und
R³ für einen über Stickstoff gebundenen 5- bis 8-gliedrigen Heterocyclus steht,
wobei der Heterocyclus substituiert ist mit 1 bis 3 Substituenten, wobei die Substituenten unabhängig voneinander ausgewählt werden aus der Gruppe bestehend aus Halogen, Hydroxy, Hydroxymethyl, Formyl, Amino, Oxo, Trifluormethyl, Trifluormethoxy, C₁-C₄-Alkyl, C₁-C₄-Alkoxy und C₁-C₄-Alkoxycarbonyl,
oder
R¹ für Phenyl steht,
wobei Phenyl substituiert ist mit 1 bis 3 Substituenten, wobei die Substituenten unabhängig voneinander ausgewählt werden aus der Gruppe bestehend aus Halogen, Cyano, Nitro, Trifluormethyl, Trifluormethoxy, Trifluormethylthio, C₁-C₄-Alkyl und C₁-C₄-Alkoxy,
R² für Phenyl steht,
wobei Phenyl substituiert sein kann mit 1 bis 3 Substituenten, wobei die Substituenten unabhängig voneinander ausgewählt werden aus der Gruppe bestehend aus Trifluormethoxy und Trifluormethylthio,
und
R³ für einen über Stickstoff gebundenen unsubstituierten 5- bis 8-gliedrigen Heterocyclus steht,
oder eines ihrer Salze, ihrer Solvate oder der Solvate ihrer Salze.

5. Verbindung nach Anspruch 4 zur Verwendung in der Behandlung und/oder Prophylaxe von Krankheiten.

6. Verwendung einer Verbindung nach Anspruch 4 zur Herstellung eines Arzneimittels zur Behandlung und/oder Prophylaxe von Krankheiten.

7. Verwendung einer Verbindung nach Anspruch 4 zur Herstellung eines Arzneimittels zur Behandlung und/oder Prophylaxe von retroviralen Erkrankungen.

8. Arzneimittel enthaltend eine Verbindung, wie in Anspruch 4 definiert, in Kombination mit einem weiteren Wirkstoff.

9. Arzneimittel enthaltend mindestens eine Verbindung nach Anspruch 4 in Kombination mit mindestens einem inerten, nichttoxischen, pharmazeutisch geeigneten Hilfsstoff.

10. Arzneimittel nach Anspruch 9 zur Verwendung in der Behandlung und/oder Prophylaxe von retroviralen Erkrankungen.

11. Antiviral wirksame Menge mindestens einer Verbindung nach Anspruch 4 oder eines Arzneimittels nach Anspruch 8 oder 9 zur Verwendung in einem Verfahren zur Bekämpfung von viralen Erkrankungen in Menschen und Tieren.

## Claims

1. Use of a compound of the formula 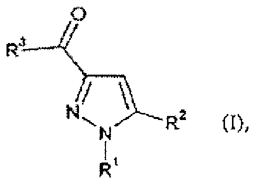 in which
R¹ stands for phenyl,
wherein phenyl is substituted by 1 to 3 substituents, wherein the substituents are independently selected from the group consisting of halogen, cyano, nitro, trifluoromethyl, trifluoromethoxy, trifluoromethyl, C₁-C₄ alkyl and C₁-C₄ alkoxy,
R² stands for phenyl,
wherein phenyl can be substituted by 1 to 3 substituents, wherein the substituents are independently selected from the group consisting of halogen, cyano, nitro, trifluoromethyl, trifluoromethoxy, trifluoromethylthio, C₁-C₄ alkyl and C₁-C₄ alkoxy,
R³ stands for a 5- to 8-membered heterocycle bound through nitrogen,
wherein the heterocycle can be substituted by 1 to 3 substituents, wherein the substituents are independently selected from the group consisting of halogen, hydroxyl, hydroxymethyl, formyl, amino, oxo, trifluoromethyl, trifluoromethoxy, C₁-C₄ alkyl, C₁-C₄ alkoxy and C₁-C₄ alkoxycarbonyl,
or one of their salts, their solvates or of the solvates of their salts,
for preparing a drug for the treatment and/or prophylaxis of retroviral diseases.

2. Use of a compound according to claim 1, **characterized in that**
R¹ stands for phenyl,
wherein phenyl is substituted by 1 to 3 substituents, wherein the substituents are independently selected from the group consisting of halogen, cyano, nitro, trifluoromethyl, trifluoromethoxy, trifluoromethylthio, C₁-C₄ alkyl and C₁-C₄ alkoxy,
R² stands for phenyl,
wherein phenyl can be substituted by 1 to 3 substituents, wherein the substituents are independently selected from the group consisting of halogen, cyano, nitro, trifluoromethyl, trifluoromethoxy, trifluoromethylthio, C₁-C₄ alkyl and C₁-C₄ alkoxy,
R³ stands for pyrrolidine-1-yl, morpholine-4-yl, thiomorpholine-4-yl, 1,3-thiazolidine-3-yl, piperidine-1-yl, piperazine-1-yl or 1,4-oxazepane-4-yl,
wherein pyrrolidine-1-yl, morpholine-4-yl, thiomorpholine-4-yl, 1,3-thiazolidine-3-yl, piperidine-1-yl, piperazine-1-yl or 1,4-oxazepane-4-yl can be substituted by 1 to 3 substituents, wherein the substituents are independently selected from the group consisting of hydroxyl, hydroxymethyl, formyl, amino, oxo, C₁-C₄ alkyl, C₁-C₄ alkoxy and C₁-C₄ alkoxycarbonyl.

3. Use of a compound according to claim 1 or 2, **characterized in that**
R¹ stands for phenyl,
wherein phenyl is substituted by 1 to 3 substituents, wherein the substituents are independently selected from the group consisting of halogen, cyano, nitro, trifluoromethyl, trifluoromethoxy, trifluoromethylthio, C₁-C₄ alkyl and C₁-C₄ alkoxy,
R² stands for phenyl,
wherein phenyl can be substituted by 1 to 3 substituents, wherein the substituents are independently selected from the group consisting of halogen, cyano, nitro, trifluoromethyl, trifluoromethoxy, trifluoromethylthio, C₁-C₄ alkyl and C₁-C₄ alkoxy,
and
R³ stands for a 5- to 8-membered heterocycle bound through nitrogen,
wherein the heterocycle is substituted by 1 to 3 substituents, wherein the substituents are independently selected from the group consisting of halogen, hydroxyl, hydroxymethyl, formyl, amino, oxo, trifluoromethyl, trifluoromethoxy, C₁-C₄ alkyl, C₁-C₄ alkoxy and C₁-C₄ alkoxycarbonyl,
or
R¹ stands for phenyl,
wherein phenol is substituted by 1 to 3 substituents, wherein the substituents are independently selected from the group consisting of halogen, cyano, nitro, trifluoromethyl, trifluoromethoxy, trifluoromethylthio, C₁-C₄ alkyl and C₁-C₄ alkoxy,
R² stands for phenyl,
wherein phenyl can be substituted by 1 to 3 substituents, wherein the substituents are independently selected from the group consisting of trifluoromethoxy and trifluoromethylthio,
and
R³ stands for an unsubstituted 5- to 8-membered heterocycle bound through nitrogen.

4. A compound of the formula 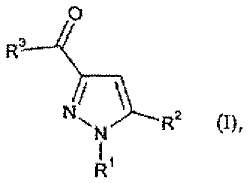 in which
R¹ stands for phenyl,
wherein phenyl is substituted by 1 to 3 substituents, wherein the substituents are independently selected from the group consisting of halogen, cyano, nitro, trifiluoromethyl, trifluoromethoxy, trifluoromethylthio, C₁-C₄ alkyl and C₁-C₄ alkoxy,
R² stands for phenyl,
wherein phenyl can be substituted by 1 to 3 substituents, wherein the substituents are independently selected from the group consisting of halogen, cyano, nitro, trifluoromethyl, trifluoromethoxy, trifluoromethylthio, C₁-C₄ alkyl and C₁-C₄ alkoxy,
and
R³ stands for a 5- to 8-membered heterocycle bound through nitrogen,
wherein the heterocycle is substituted by 1 to 3 substituents, wherein the substituents are independently selected from the group consisting of halogen, hydroxyl, hydroxymethyl, formyl, amino, oxo, trifluoromethyl, trifiiuoromethoxy, C₁-C₄ alkyl, C₁-C₄ alkoxy and C₁-C₄ alkoxycarbonyl,
or
R¹ stands for phenyl,
wherein phenyl is substituted by 1 to 3 substituents, wherein the substituents are independently selected from the group consisting of halogen, cyano, nitro, trifluoromethyl, trifluoromethoxy, trifluoromethylthio, C₁-C₄ alkyl and C₁-C₄ alkoxy,
R² stands for phenyl,
wherein phenyl can be substituted by 1 to 3 substituents, wherein the substituents are independently selected from the group consisting of trifluoromethoxy and trifluoromethylthio,
and
R³ stands for an unsubstituted 5- to 8-membered heterocycle bound through nitrogen,
or one of their salts, their solvates or of the solvates of their salts.

5. The compound according to claim 4 for use in the treatment and/or prophylaxis of diseases.

6. The use of a compound according to claim 4 for preparing a drug for the treatment and/or prophylaxis of diseases.

7. The use of a compound according to claim 4 for preparing a drug for the treatment and/or prophylaxis of retroviral diseases.

8. A drug containing a compound as defined in claim 4, in combination with a further active substance.

9. A drug containing at least one compound according to claim 4 in combination with at least one inert, nontoxic, pharmaceutically suitable excipient.

10. The drug according to claim 9 for use in the treatment and/or prophylaxis of retroviral diseases.

11. Antivirally effective amount of at least one compound according to claim 4 or of a drug according to claim 8 or 9 for use in the treatment of viral diseases in humans and animals.

## Revendications

1. Utilisation d'un composé de la formule 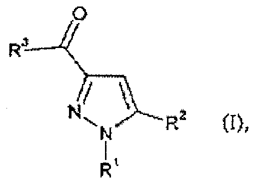 dans laquelle
R¹ représente le phényle,
le phényle étant substitué par 1 à 3 substituants, étant donné que les substituants sont sélectionnés indépendamment l'un de l'autre dans le groupe constitué par les éléments halogène, cyano, nitro, trifluorométhyle, trifluorométhoxy, trifluorométhylthio, C₁-C₄ alkyle et C₁-C₄ alcoxy,
R² représente le phényle,
le phényle pouvant être substitué par 1 à 3 substituants, étant donné que les substituants sont sélectionnés indépendamment l'un de l'autre dans le groupe constitué par les éléments halogène, cyano, nitro, trifluorométhyle, trifluorométhoxy, trifluorométhylthio, C₁-C₄ alkyle et C₁-C₄ alcoxy,
R³ représente un hétérocycle de 5 à 8 éléments lié par l'azote,
l'hétérocycle pouvant être substitué par 1 à 3 substituants, étant donné que les substituants sont sélectionnés indépendamment l'un de l'autre dans le groupe constitué par les éléments halogène, hydroxyle, hydroxyméthyle, formyle, amino, oxo, trifluorométhyle, trifluorométhoxy, C₁-C₄-alkyle, C₁-C₄ alcoxy et C₁-C₄ alcoxycarbonyle,
ou un de ses sels, ses solvates ou des solvates de ses sels,
pour la préparation d'un médicament pour le traitement et/ou la prophylaxie de maladies rétrovirales.

2. Utilisation d'un composé selon la revendication 1, **caractérisée en ce que**
R¹ représente le phényle,
le phényle étant substitué par 1 à 3 substituants, étant donné que les substituants sont sélectionnés indépendamment l'un de l'autre dans le groupe constitué par les éléments halogène, cyano, nitro, trifluorométhyle, trifluorométhoxy, trifluorométhylthio, C₁-C₄ alkyle et C₁-C₄ alcoxy,
R² représente le phényle,
le phényle pouvant être substitué par 1 à 3 substituants, étant donné que les substituants sont sélectionnés indépendamment l'un de l'autre dans le groupe constitué par les éléments halogène, cyano, nitro, trifluorométhyle, trifluorométhoxy, trifluorométhylthio, C₁-C₄ alkyle et C₁-C₄ alcoxy,
R³ représente la pyrrolidine-1-yl, morpholine-4-yl, thiomorpholine-4-yl, 1,3-thiazolidine-3-yl, pipéridine-1-yl, pipérazine-1-yl ou 1,4-oxazépane-4-yl,
l'hétérocycle pouvant être substitué par 1 à 3 substituants, étant donné que les substituants sont sélectionnés indépendamment l'un de l'autre dans le groupe constitué par les éléments halogène, hydroxyle, hydroxyméthyle, formyle, amino, oxo, trifluorométhyle, trifluorométhoxy, C₁-C₄-alkyle, C₁-C₄ alcoxy et C₁-C₄ alcoxycarbonyle.

3. Utilisation d'un composé selon la revendication 1 ou 2, **caractérisée en ce que**
R¹ représente le phényle,
le phényle étant substitué par 1 à 3 substituants, étant donné que les substituants sont sélectionnés indépendamment l'un de l'autre dans le groupe constitué par les éléments halogène, cyano, nitro, trifluorométhyle, trifluorométhoxy, trifluorométhylthio, C₁-C₄ alkyle et C₁-C₄ alcoxy,
R² représente le phényle,
le phényle pouvant être substitué par 1 à 3 substituants, étant donné que les substituants sont sélectionnés indépendamment l'un de l'autre dans le groupe constitué par les éléments halogène, cyano, nitro, trifluorométhyle, trifluorométhoxy, trifluorométhylthio, C₁-C₄ alkyle et C₁-C₄ alcoxy,
et
R³ représente un hétérocycle de 5 à 8 éléments lié par l'azote,
l'hétérocycle étant substitué par 1 à 3 substituants, étant donné que les substituants sont sélectionnés indépendamment l'un de l'autre dans le groupe constitué par les éléments halogène, hydroxyle, hydroxyméthyle, formyle, amino, oxo, trifluorométhyle, trifluorométhoxy, C₁-C₄-alkyle, C₁-C₄ alcoxy et C₁-C₄ alcoxycarbonyle,
ou
R¹ représente le phényle,
le phényle étant substitué par 1 à 3 substituants, étant donné que les substituants sont sélectionnés indépendamment l'un de l'autre dans le groupe constitué par les éléments halogène, cyano, nitro, trifluorométhyle, trifluoraméthoxy, trifluorométhylthio, C₁-C₄ alkyle et C₁-C₄ alcoxy,
R² représente le phényle,
le phényle pouvant être substitué par 1 à 3 substituants, étant donné que les substituant sont sélectionnés indépendamment l'un de l'autre dans le groupe constitué par les éléments trifluorométhoxy et trifluorométhylthio,
et
R³ représente un hétérocycle non-substitué de 5 à 8 éléments lié par l'azote,

4. Composé de la formule 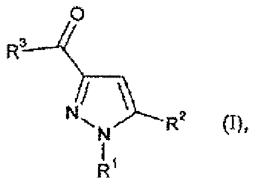 dans laquelle
R¹ représente le phényle,
le phényle étant substitué par 1 à 3 substituants, étant donné que les substituant sont sélectionnés indépendamment l'un de l'autre dans le groupe constitué par les éléments halogène, cyano, nitro, trifluorométhyle, trifluorométhoxy, trifluorométhylthio, C₁-C₄ alkyle et C₁-C₄ alcoxy,
R² représente le phényle,
le phényle pouvant être substitué par 1 à 3 substituants, étant donné que les substituants sont sélectionnés indépendamment l'un de l'autre dans le groupe constitué par les éléments halogène, cyano, nitro, trifluorométhyle, trifluorométhoxy, trifluorométhylthio, C₁-C₄ alkyle et C₁-C₄ alcoxy,
et
R³ représente un hétérocycle de 5 à 8 éléments lié par l'azote,
l'hétérocycle étant substitué par 1 à 3 substituants, étant donné que les substituants sont sélectionnés indépendamment l'un de l'autre dans le groupe constitué par les éléments halogène, hydroxyle, hydroxyméthyle, formyle, amino, oxo, trifluorométhyle, trifluorométhoxy, C₁-C₄-alkyle, C₁-C₄ alcoxy et C₁-C₄ alcoxycarbonyle,
ou
R¹ représente le phényle,
le phényle étant substitué par 1 à 3 substituants, étant donné que les substituants sont sélectionnés indépendamment l'un de l'autre dans le groupe constitué par les éléments halogène, cyano, nitro, trifluorométhyle, trifluorométhoxy, trifluorométhylthio, C₁-C₄ alkyle et C₁-C₄ alcoxy,
R² représente le phényle,
le phényle pouvant être substitué par 1 à 3 substituants, étant donné que les substituants sont sélectionnés indépendamment l'un de l'autre dans le groupe constitué par les éléments trifluorométhoxy et trifluorométhylthio,
et
R³ représente un ilëtérocycle non-substitué de 5 à 8 éléments lié par l'azote,
ou un de ses sels, ses solvates ou des solvates de ses sels.

5. Composé selon la revendication 4 pour utilisation dans le traitement et/ou la prophylaxie de maladies.

6. Utilisation d'un composé selon la revendication 4 pour la préparation d'un médicament pour le traitement et/ou la prophylaxie de maladies.

7. Utilisation d'un composé selon la revendication 4 pour la préparation d'un médicament pour le traitement et/ou la prophylaxie de maladies rétrovirales.

8. Médicament contenant un composé tel que défini à la revendication 4 en combinaison avec une autre substance active.

9. Médicament contenant au moins un composé selon la revendication 4 en combinaison avec au moins un excipient inerte, non-toxique, pharmaceutiquement approprié.

10. Médicament selon la revendication 9 pour utilisation dans le traitement et/ou la prophylaxie de maladies rétrovirales.

11. Quantité à effet antiviral d'au moins un composé selon la revendication 4 ou d'un médicament selon la revendication 8 ou 9 pour utilisation dans un procédé de lutte contre les maladies virales chez les êtres humains et les animaux.
